# EUROPEAN PATENT SPECIFICATION

(11) **EP 4 358 856 B1**
(45) Date of publication and mention of the grant of the patent: **23.09.2026**
(21) Application number: 22732839.0
(22) Date of filing: 31.05.2022
(51) Int. Cl.: A61B 8/12, A61B 17/34, A61M 25/06

(54) **MEDICAL LEAD SYSTEM**
MEDIZINISCHES LEITUNGSSYSTEM
SYSTÈME DE DÉRIVATION MÉDICALE

(30) Priority: 22.06.2021 US 202163213674 P; 24.05.2022 US 202217664787
(43) Date of publication of application: 01.05.2024
(73) Proprietor: Medtronic, Inc., Minneapolis, Minnesota 55432 (US)
(72) Inventor: SEIFERT, Kevin R., Minneapolis, Minnesota 55432 (US); WHITMAN, Teresa A., Minneapolis, Minnesota 55432 (US); BAXTER, Jonathan E., Minneapolis, Minnesota 55432 (US); MARSHALL, Mark T., MINNEAPOLIS, Minnesota 55432 (US); HUGHES, Jonathan A., Minneapolis, Minnesota 55432 (US)
(74) Representative: Zimmermann & Partner Patentanwälte mbB
(86) International application number: PCT/US2022/031532
(87) International publication number: WO 2022/271416

(56) References cited:
- WO-A1-2015/123313
- WO-A1-2018/081239
- WO-A1-2019/023609
- WO-A1-2020/036886

## Description

### TECHNICAL FIELD

This disclosure is related to an implantable medical systems, such as a medical lead for implantation in a heart.

### BACKGROUND

Implantable medical leads may be adapted to treat a wide variety of cardiac dysfunctions. Implantable medical leads include electrodes and/or other elements for physiological sensing and/or therapy delivery. Implantable medical leads allow the sensing/therapy elements to be positioned at one or more target locations for those functions. The implantable medical lead may be navigated through vasculature of a patient to reach the target locations. An electrode supported by the medical lead may establish electrical communication with tissues of the heart to sense cardiac signals generated by the heart and/or deliver cardiac pacing to the patient. Evaluation of the target location is often conducted to determine a satisfactory location for the electrode within the heart in order to provide adequate activation of the cardiac system. WO 2019/023609 A1 relates to a directional balloon transseptal insertion device for medical procedures.

### SUMMARY

This disclosure describes a medical lead system configured to implant an electrode within tissue of a patient. The medical lead system includes a lead body mechanically supporting a fixation member configured to extend distally from the lead body. An inflatable balloon affixed to the lead body is configured to inflate and extend distally beyond a distal end of the lead body, such that the inflated balloon substantially surrounds at least a portion of the fixation member. The balloon is configured to define a distal cavity radially displacing outward away from the fixation member when the balloon defines the distal cavity. In some examples, the balloon is configured to extend distally beyond the fixation member when the balloon defines the distal cavity. In some examples, the fixation member is configured to extend distally beyond the balloon when the balloon defines the distal cavity. In examples, the medical lead system includes an electrode mechanically supported by the fixation member and configured to electrically communicate with the tissue when the balloon defines the distal cavity. The balloon is configured to substantially re-establish a deflated configuration to allow implantation of the fixation member and securing of the lead body to the tissue.

In an example, a medical lead system comprises: an elongated lead body including a distal end, wherein the lead body is configured to extend through vasculature of a patient; a fixation member configured to extend distal to the distal end; and a balloon affixed to an exterior surface of the lead body and defining a deflated configuration and an inflated configuration, wherein the balloon is configured to expand radially outward from the exterior surface and extend distally beyond the distal end of the lead body when the balloon inflates from the deflated configuration to the inflated configuration, wherein a portion of an outer surface of the balloon is configured to define a distal cavity surrounding at least some portion of the fixation member when the balloon expands radially outward, and wherein the portion of the outer surface is configured to flare radially away from the fixation member as the portion of the outer surface extends distally beyond the distal end of the lead body.

In an example, a medical lead system comprises: an elongated lead body including a distal end, wherein the lead body is configured to extend through vasculature of a patient, wherein the lead body defines a longitudinal axis extending through the distal end; a fixation member configured to extend distal to the distal end of the lead body, wherein the longitudinal axis extends through at least a portion of the fixation member; and a balloon affixed to an exterior surface of the lead body and defining a deflated configuration and an inflated configuration, wherein the balloon is configured to expand radially outward from the exterior surface and extend distally beyond the distal end of the lead body when the balloon inflates from the deflated configuration to the inflated configuration, wherein a portion of an outer surface of the balloon is configured to define a distal cavity surrounding at least some portion of the fixation member when the balloon expands radially outward, wherein the portion of the outer surface is configured to flare radially away from the fixation member as the portion of the outer surface extends distally beyond the distal end of the lead body, and wherein the portion of the outer surface is configured to increase a radial displacement from the longitudinal axis to the portion of the outer surface when the portion of the outer surface flares radially away from the fixation member.

In an example, a method comprises: inflating, with an inflating medium, a balloon affixed to an exterior surface of the lead body, wherein the lead body includes a distal end, and wherein the lead body is configured to extend through vasculature of a patient, and wherein the balloon is configured to expand radially outward from the exterior surface and extend distally beyond the distal end when the balloon inflates from a deflated configuration to an inflated configuration; and defining, using a portion of an outer surface of the balloon, a distal cavity surrounding at least some portion of the fixation member when the balloon expands radially outward, wherein the portion of the outer surface flares radially away from the fixation member as the portion of the outer surface extends distally beyond the distal end of the lead body.

The details of one or more examples are set forth in the accompanying drawings and the description below. Other features, objects, and advantages will be apparent from the description and drawings, and from the claims.

### BRIEF DESCRIPTION OF DRAWINGS

FIG. 1 is a conceptual diagram illustrating an example medical lead system accessing a target site within a patient.
FIG. 2 is a schematic illustration of the example medical lead system within a sheath.
FIG. 3 is a schematic illustration of the example medical lead system positioned distal to the sheath.
FIG. 4 is a schematic illustration of the example medical lead system with a balloon in an inflated configuration.
FIG. 5 is a schematic illustration of the example medical lead system in the vicinity of a tissue wall.
FIG. 6 is a schematic illustration of the example medical lead system in the vicinity of a target site on the tissue wall.
FIG. 7 is a schematic illustration of the example medical lead system of with the balloon in a deflated configuration.
FIG. 8 is a schematic illustration of the example medical lead system secured to the tissue wall using a fixation member.
FIG. 9 is a schematic illustration of the example medical lead system in the vicinity of a tissue wall, with a fixation element proximal to the balloon,
FIG. 10 is a schematic illustration of the example medical lead system in the vicinity of a target site on the tissue wall, with the fixation element proximal to the balloon.
FIG. 11 is a schematic cross-sectional illustration of the example medical lead system with the balloon in the deflated configuration.
FIG. 12 is a schematic cross-sectional illustration of the example medical lead system with the balloon in the inflated configuration and the fixation element distal to the balloon.
FIG. 13 is a schematic cross-sectional illustration of the example medical lead system with the balloon in the inflated configuration and the fixation element proximal to the balloon.
FIG. 14 is a schematic cross-sectional illustration of a distal balloon portion in the deflated configuration with a fixation element in a first position relative to the balloon.
FIG. 15 is a schematic cross-sectional illustration of the distal balloon portion in the inflated configuration with the fixation element in the first position relative to the balloon.
FIG. 16 is a schematic cross-sectional illustration of a distal balloon portion in the deflated configuration with a fixation element in a second position relative to the balloon.
FIG. 17 is a schematic cross-sectional illustration of the distal balloon portion in the inflated condition with the fixation element in the second position relative to the balloon.
FIG. 18 is a schematic illustration of a proximal balloon portion defining a first inner dimension and a second inner dimension.
FIG. 19 is a schematic illustration of a proximal balloon portion defining a first thickness and a second thickness.
FIG. 20 is a schematic cross-sectional illustration of the example medical lead system illustrating first and second fixation structures.
FIG. 21 is a schematic cross-sectional illustration of the example medical lead system illustrating a first balloon and a second balloon.
FIG. 22 illustrates an example technique for using the example medical lead system.

### DETAILED DESCRIPTION

This disclosure describes a medical lead system configured to implant an electrode within tissue of a patient, such as a septal wall of the heart. The medical lead system includes a lead body mechanically supporting a fixation member such as a helix configured to extend distally from the lead body. The medical lead system further includes an inflatable balloon configured to inflate from a deflated configuration and extend distally beyond a distal end of the lead body, such that the inflated balloon substantially surrounds a portion of the fixation member to minimize and/or eliminate physical interference between the fixation member and anatomical structures within the patient. The inflated balloon is configured to define a distal cavity surrounding at least some portion of the fixation element. The distal cavity flares radially away from the fixation element as the balloon extends distally beyond the distal end of the lead body when the balloon is in the inflated configuration. In examples, the inflated balloon is configured to extend distally beyond the fixation member. In examples, the fixation member is configured to extend distally beyond the balloon when the balloon is in the inflated configuration. An electrode mechanically supported by the fixation member may electrically communicate with the tissue (e.g., for pace mapping) when the distal portion of the lead body is in the vicinity of the tissue wall. The balloon is configured to substantially re-establish the deflated configuration to allow implantation of the fixation member (e.g., based on the pace mapping) and securing of the lead body to the tissue.

The medical lead system is configured to transit through vasculature of the patient to position the distal portion of the lead body in the vicinity of a target area, such as an area within a chamber of the heart. For example, the medical lead system may be configured to allow a clinician to navigate the lead body through a vein of the heart (e.g., an innominate vein, an interior vena cava (IVC), and/or a superior vena cava (SVC)) to a target location within a right ventricle (RV), right atrium (RA), or another area of the heart. In examples, the medical lead system is configured such that the balloon may be inflated within a vein of the heart enroute to the target area to, for example, minimize and/or eliminate physical interference between the fixation member and other anatomical structures as the medical lead system is transited within the patient.

The balloon is configured to expand radially outward from the lead body when the balloon is inflated from the deflated configuration to the inflated configuration. The balloon may be configured to define a substantially toroidal shape around the lead body in the inflated configuration such that, for example, the lead body extends through the toroid hole. In examples, the fixation member is configured to extend through the toroid hole. In some examples, the balloon is configured to expand around the distal portion of the lead body such that a blood flow imparts a force on the balloon in the direction of the blood flow (e.g., such that the balloon acts as a "sail"). The balloon may be configured to transmit the force to the lead body to assist in transiting the lead body through the vasculature and/or a heart chamber of the patient while substantially shrouding the fixation member. In examples, the balloon is configured to inflate to an expanded dimension (e.g., a diameter) of at least five times a diameter of the lead body. In examples, the lead body defines an inner lumen configured to deliver an inflating medium to cause inflation of the balloon.

The balloon may be configured to expand asymmetrically from the deflated configuration to the inflated configuration in order to cause the balloon to expand distally beyond the distal end of the lead body ("lead body distal end"). In examples, the balloon includes a balloon body affixed to an exterior surface of the lead body at a first location and a second location distal to the first location. The balloon body may define a proximal balloon portion adjacent to the first location and a distal balloon portion adjacent the second location. The balloon may be configured such that the proximal balloon portion and the distal balloon portion are both substantially between the first location and the second location when the balloon is in the deflated configuration. In examples, the balloon is configured to cause the distal balloon portion to extend distal to the second location as the proximal balloon portion remains substantially between the first location and the second location during balloon inflation, such that the balloon expands asymmetrically to extend distally beyond the distal tip of the lead body in the inflated configuration.

In examples, the balloon defines an interior volume configured to contain the inflating medium when the balloon inflates from the deflated configuration to the inflated configuration. The interior volume may be fluidly coupled to the inner lumen defined by the lead body. The balloon may define an interior surface area configured to contact the inflating medium when the balloon inflates. In examples, the balloon is configured to cause the distal balloon portion to extend distally based on altering the interior surface area as the balloon body extends over the lead body in the deflated configuration. For example, the balloon may define a first surface area comprising the interior surface area within the proximal balloon portion and a second surface area comprising the interior surface area with the distal balloon portion. In examples, the first surface area may be greater than the second surface area, such that inflating medium exerts a greater force against the proximal balloon portion than the distal balloon portion when the inflating medium flows into the interior volume. The greater force on the proximal balloon portion may substantially cause the distal balloon portion to "roll" distally and extend over the lead body distal end as the inflation continues.

The medical lead system may be configured to electrically communicate with tissue within a patient when the balloon is in the inflated configuration. In examples, the fixation member mechanically supports an electrode configured to electrically communicate with the tissue. The fixation member may be configured to position the electrode to communicate with the tissue when the inflated balloon extends distal to the fixation member or when the fixation member extends distal to the inflated balloon. In some examples, the fixation member is configured to allow the electrode to contact the tissue when the fixation member extends distal to the inflated balloon. The medical lead system may include a conductor in electrical communication with the electrode. In examples, the medical lead system includes processing circuitry configured to deliver therapy to the tissue using the conductor and the electrode. The medical lead system may be configured such that a clinician can perform cardiac mapping using the electrode while the balloon is inflated, such that the cardiac mapping may occur as the balloon substantially shrouds the fixation member to reduce and/or eliminate physical interferences between the fixation member and surrounding anatomical structures.

The balloon is configured to deflate from the inflated configuration to substantially re-establish the deflated configuration. For example, a clinician may cause the balloon to substantially re-establish the deflated configuration (e.g., by venting the balloon through the inner lumen) in order to secure the medical lead system to the tissue using the fixation member. In examples, the fixation member defines a helix extending distal to the lead body distal end and at least partially shrouded by the inflated balloon. The clinician may deflate the balloon via the inner lumen to cause the balloon to deflate and withdraw proximal to the lead body distal end, such that the fixation member is substantially unshrouded. The clinician may then cause the helix to substantially fully engage the tissue by, for example, torquing the lead body around a longitudinal axis to drive the helix into a surface of the tissue. The balloon may be configured to remain affixed to the lead body and proximal to the fixation member and/or electrode in the deflated configuration, such that processing circuitry may deliver therapy to the patient via the electrode with the balloon in the deflated configuration.

In examples, the medical lead system may include a sheath (e.g., an introducer sheath and/or delivery catheter) configured to deliver the lead body to the vasculature or other areas within the patient. The sheath may define a sheath lumen and a sheath opening at a distal end. The medical lead system may be configured such that the lead body may be transited through the lumen and through the sheath opening when the balloon is in the deflated configuration. In examples, the lead body includes a marker (e.g., proximal to the balloon) configured to indicate the balloon is distal to the sheath opening as the lead body is distally translated in the sheath lumen and through the sheath opening. The marker may be configured to configured to be visible on an imaging system, such as a fluoroscope, ultrasound, or other systems configured to provide images of anatomy within a patient. The marker may serve as an indication to a clinician that the balloon is no longer surrounded by the sheath lumen and may be inflated from the deflated configuration to the inflated configuration.

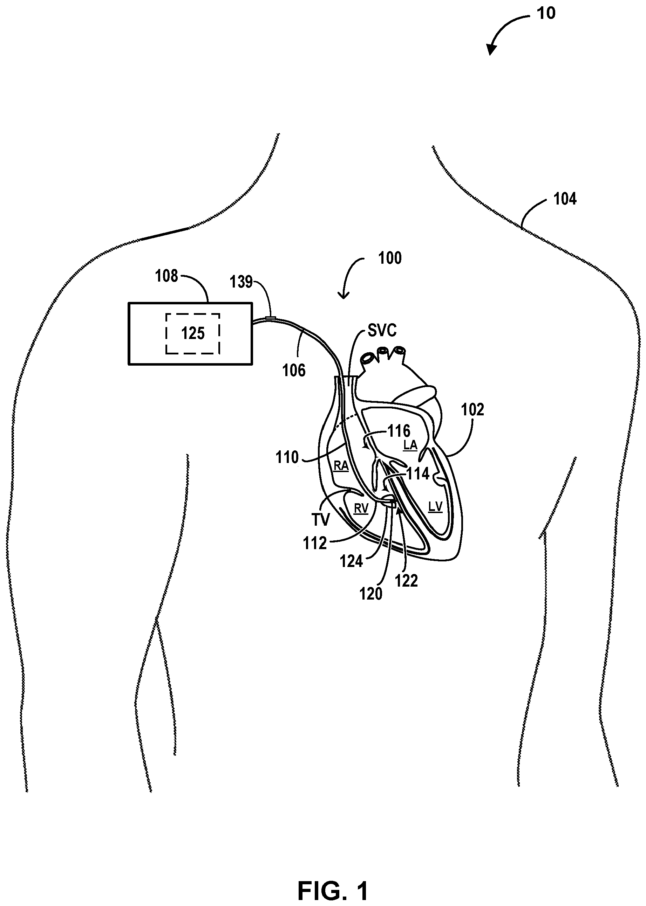

FIG. 1 is a conceptual diagram illustrating a portion of an example medical system 100 configured to deliver therapy (e.g., pacing) to a heart 102 of a patient 104. Medical system 100 includes medical lead 106 extending from a medical device 108 through vasculature of patient 104. Medical lead 106 includes an elongated lead body 110 with a distal portion 112 of lead body 110 ("lead body distal portion 112") generally positioned at a target site 114 within a patient 104. In examples, as illustrated in FIG. 1, target site 114 is a region in the ventricular septal wall of heart 102. In examples, medical lead 106 may be oriented such that lead body distal portion 112 positions at another portion of heart 102. For example, implantable medical lead may be oriented such that lead body distal portion 112 generally positions at a target site 116 in the atrioventricular septal wall. Medical system 100 may include additional leads coupled to medical device 108 and extending into heart 102.

Lead body distal portion 112 includes a distal end 120 ("lead distal end 120") and a fixation member 122 configured to extend distally beyond lead distal end 120. Lead body distal portion 112 mechanically supports fixation member 122. Fixation member 122 is configured to penetrate tissue of the patient 104 at or near a target sites, such as target sites 114, 116. For example, fixation member 122 may be configured to penetrate cardiac tissue of a septal wall in a RV, RA, LV, and/or LA of heart 102, or penetrate cardiac tissue in another area of heart 102. In examples, fixation member 122 is configured to remain substantially stationary with respect to lead distal end 120, such that fixation member 122 is substantially fixed in place on lead body distal portion 112. In other examples, fixation member 122 may be configured to translate relative to lead distal end 120. For example, fixation member 122 may be configured to translate distally or proximally within a lumen defined by lead body distal portion 112. Fixation member 122 may have various shapes such as helices, tines, screws, rings, and so on.

In examples, fixation member 122 mechanically supports an electrode (not shown) configured to electrically communicate with tissue when fixation member 122 positions the electrode in proximity to target sites 114, 115. In examples, the electrode is configured to provide pacing to heart 102. The electrode may be electrically connected to one or more conductors (not shown) extending through lead body 110. In some examples, the conductors are electrically connected to circuitry 125 of medical device 108, with circuitry 125 configured to deliver therapy signals to and/or sense cardiac signals from the electrode using the conductor. Fixation member 122 may be configured to position the electrode such that the electrode conducts the electrical signals to the target tissue of heart 102, causing the cardiac muscle, e.g., of the ventricles, to depolarize and, in turn, contract at a regular interval.

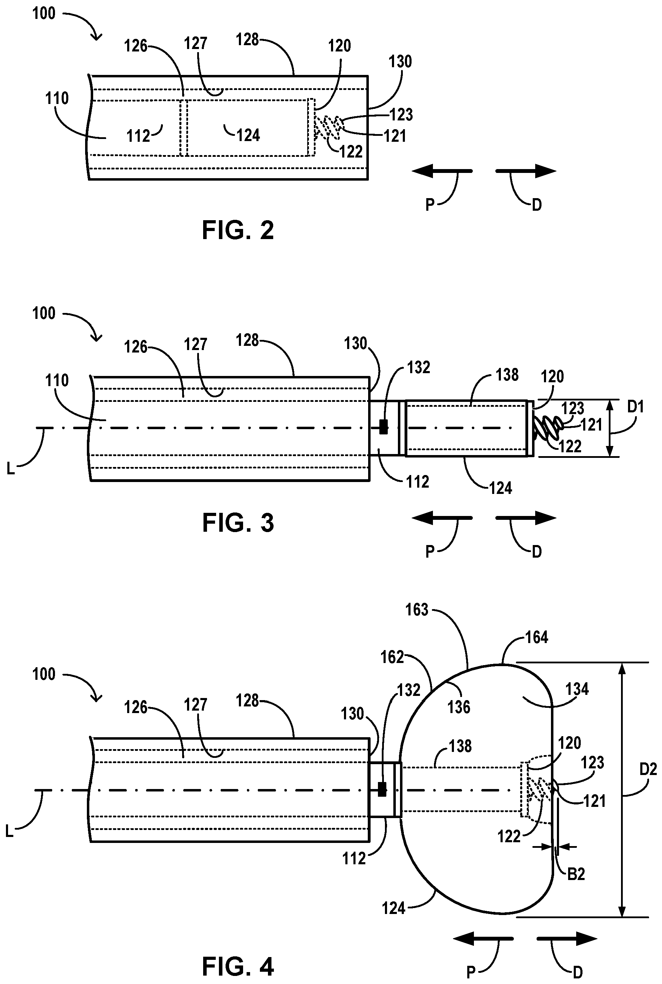

Medical system 100 includes a balloon 124 attached to an exterior surface of lead body distal portion 112. Balloon 124 defines an interior volume configured to receive an inflating medium (e.g., air, saline, or another medium) and cause inflation of balloon 124. In examples, lead body 110 defines a lumen fluidly coupled to the interior volume and configured such that a clinician may deliver the inflating medium to the interior volume defined by balloon 124. Balloon 124 is configured to inflate from a deflated configuration defining an initial dimension (e.g., an initial diameter) and expand radially outward from lead body distal portion 112 to an inflated configuration defining an expanded dimension (e.g., an expanded diameter). Balloon 124 is depicted in the inflated configuration in FIG. 1. Balloon 124 is configured to extend distally beyond lead distal end 120 when balloon 124 is in the inflated configuration. In examples, balloon 124 defines a substantially toroidal shape surrounding lead body distal portion 112 and lead distal end 120 when balloon 124 is in the inflated configuration. In some examples, balloon 124 is configured to extend distal to fixation member 122 (e.g., fixation member distal end 121 (FIGS. 3-17)) when balloon 124 is in the inflated configuration. In some examples, fixation member 122 is configured to extend distal to balloon 124 when balloon 124 is in the inflated configuration. Balloon 124 may be configured to substantially surround a portion of fixation member 122 in the inflated configuration in order to, for example, minimize and/or eliminate physical interference between fixation member 122 and other anatomical structures within the patient.

A clinician may maneuver lead body 110 through the vasculature of patient 104 in order to position lead body distal portion 112 at or near a target site, such as target site 114, 116. Medical system 100 may be configured to allow a clinician to maneuver lead body 110 through the vasculature when balloon 124 is in the inflated configuration. For example, with balloon 124 in the inflated configuration, the clinician may guide lead body distal portion 112 through the superior vena cava (SVC), into the RA, and past tricuspid valve (TV) into the RV in order to access target site 114 on the atrioventricular septal wall. The inflated balloon 124 may substantially surround a portion of fixation member 122 to maintain at least a radial displacement between fixation member 122 and anatomical structures within patient 104 (e.g., the TV, or other structures within heart 102) during the transit of lead body distal portion 112. Medical system 100 may be configured to accommodate other pathways or techniques to reach target sites within patient 104 with balloon 124 in the inflated configuration. For example, medical system 100 may be configured such that the inflated configuration accommodates transit through an innominate vein, an interior vena cava (IVC), and/or another veinous pathway enroute to a chamber of heart 102.

In examples, the radial expansion of balloon 124 around the lead body distal portion 112 distal portion causes a blood flow to impart a force on balloon 124 in the direction of the blood flow. Balloon 124 may thus substantially act as a sail, assisting the clinician during a transit to target site 114, 116. Balloon 124 may transmit the force imparted to lead body distal portion 112 to assist in transiting lead body distal portion 112 through vasculature of patient 104 and/or a heart 102 while substantially shrouding fixation member 122. For example, in the inflated configuration, blood flow from the RA to the RV of heart 102 may impart a force on balloon 124 which assists the transit of lead body distal portion 112 through the TV of heart 102. Balloon 124 may transmit the force to lead body distal portion 112 to assist transition of lead body distal portion 112 through the TV while substantially shrouding fixation member 122 to minimize and/or eliminate physical interference between fixation member 122 and the TV.

Medical system 100 is configured to conduct mapping (e.g., electrophysiological mapping) of regions of heart 102 to locate an initial target site (e.g., target site 114, 116). In examples, medical system 100 is configured to conduct the mapping when balloon 124 is in the inflated configuration. Fixation member 122 may be configured to position the mechanically supported electrode in sufficient proximity to tissue of heart 102 such that the electrode may establish electrical communication with the tissue for conduct of the mapping. Medical system 100 may be configured to conduct contact-based and/or substantially contact-less pace mapping of heart 102 using the electrode. With balloon 124 in the inflated configuration, a clinician may navigate lead body distal portion 112 to potential implantation sites (e.g., target site 114, 116) and evaluate the adequacy of each potential site through observation of a signal (e.g., ECG and/or EGM signals) generated by heart 102 as the electrode electrically communicates with the tissue. When the clinician determines medical system 100 has located an adequate target site, the clinician may deflate balloon 124 to allow insertion of fixation member 122 at the target site. Balloon 124 may substantially re-establish the deflated configuration when the clinician causes balloon 124 to deflate.

In some examples, medical system 100 is configured to monitor a pressure within an interior volume (e.g., interior volume 134 (FIGS. 4-6)) defined by balloon 124. For example. Medical system 100 may include a pressure sensor configured to sense a pressure within the interior volume and communicate a signal indicative of the pressure to processing circuitry of an output device. The medical lead system may be configured such that a clinician may monitor the pressure as lead body 110 is transited through the vasculature of patient 104 and/or chambers of heart 102 in order to provide an indication of the location of lead body 110 within patient 104. For example, the signal indicative of the pressure may provide an indication that lead body 110 has entered a chamber of heart 102.

FIG. 2 is a conceptual diagram of medical system 100 in a configuration which might be utilized to deliver lead body 110 to vasculature or other areas within patient 104 enroute to positioning lead body distal portion 112 in the vicinity of a target site. FIG. 2 illustrates lead body 110 positioned within a sheath lumen 126 of a sheath 128. Sheath 128 may be, for example, an introducer sheath, such an introducer sheath configured to provide access to a jugular, innominate, and/or subclavian vein. In some examples, sheath 128 is a delivery catheter. Sheath 128 includes an inner wall 127 defining sheath lumen 126 and further includes a sheath opening 130 to sheath lumen 126. Balloon 124 is affixed to lead body distal portion 112. Ffixation member 122 extends distal (e.g., in the distal direction D) to lead distal end 120 of lead body distal portion 112. Iinner wall 127, lead body distal portion 112, lead distal end 120, balloon 124, and fixation member 122 are depicted in dashed lines within sheath lumen 126. FIG. 2 illustrates medical system 100 with balloon 124 in the deflated configuration. Medical system 100 is configured to translate through sheath lumen 126 to pass through sheath opening 130 when balloon 124 is in the deflated configuration. Fixation member 122 mechanically supports an electrode 123 configured to electrically communicate with tissue when positioned in the vicinity of a target site within patient 104, such as target site 114, 116.

FIG. 3 is a conceptual diagram of medical system 100 in a configuration which might be utilized to position balloon 124 outside of sheath lumen 126, such that balloon 124 may be expanded enroute to positioning lead body distal portion 112 in the vicinity of a target site within patient 104. FIG. 3 illustrates balloon 124 in the deflated configuration and defining an maximum initial dimension D1 (e.g., a diameter) perpendicular to a longitudinal axis L defined by lead body 110. Medical system 100 defines maximum initial dimension D1 to allow lead body 110 to translate through sheath lumen 126 and sheath opening 130. In examples, the lead body 110 includes a marker 132 (e.g., proximal to balloon 124) configured to indicate that balloon 124 is distal to sheath opening 130, such that balloon 124 is free to expand without constraint by sheath lumen 126 (e.g., inner wall 127). Marker 132 may be configured to configured to be visible on an imaging system, such as a fluoroscope, ultrasound, or other systems configured to provide images of medical system 100 within patient 104.

FIG. 4 is a conceptual diagram of medical system 100 with balloon 124 in the inflated configuration. Balloon 124 defines an interior volume 134 configured to contain an inflating medium (e.g., air, saline, or another inflating medium) to cause balloon 124 to transition from the deflated configuration of FIG. 2 and FIG. 3 to the inflated configuration depicted in FIG. 4. In examples, interior volume 134 is bound at least in part by an inner surface 136 of balloon 124 ʺʺballoon inner surface 136") and an exterior surface 138 of lead body distal portion 112 ("distal exterior surface 138"). Lead body 110 may define an inflation lumen configured to provide the inflating medium to interior volume 134.

In the inflated configuration, balloon 124 defines an maximum expanded dimension D2 (e.g., a diameter) perpendicular to longitudinal axis L. The maximum expanded dimension D2 of the inflated configuration is greater than the maximum initial dimension D1 of the deflated configuration. In the inflated configuration, balloon 124 extends distal to lead distal end 120, with a portion of fixation member 122 extending distal to balloon 124. Balloon 124 may substantially surround longitudinal axis L in the inflated configuration, such that balloon 124 substantially forms a bumper circumferentially around fixation member 122. In examples, balloon 124 defines a substantially toroidal shape surrounding lead body distal portion 112 and lead distal end 120 when balloon 124 is in the inflated configuration. Balloon 124 may be configured such that lead body distal portion 112 extends at least partially within a hole defined by the substantially toroidal shape. In examples, fixation member 122 is configured to extend at least partially through the hole defined by the substantially toroidal shape.

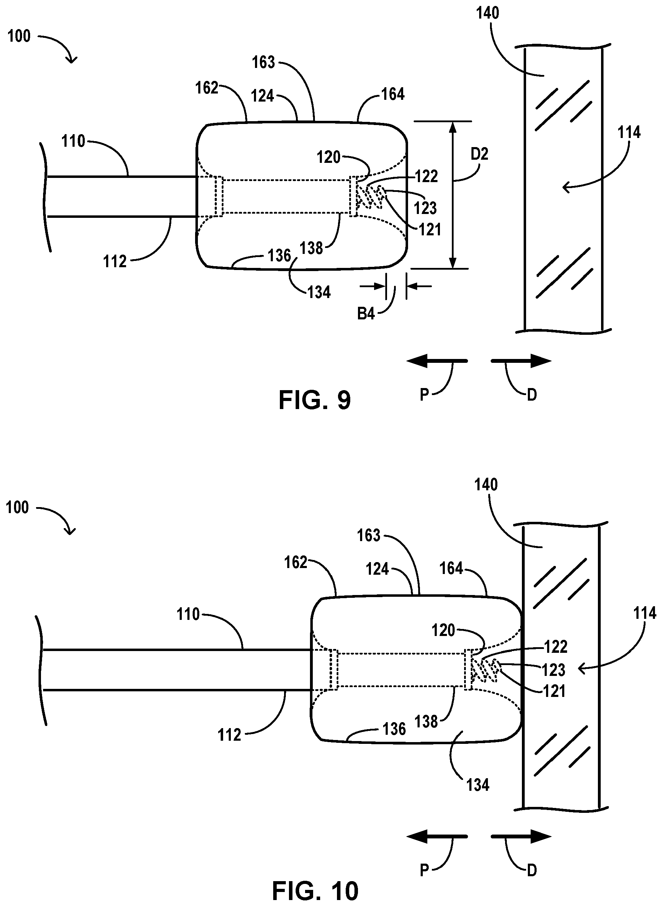

In some examples, fixation member 122 is configured to extend distal to balloon 124 when balloon 124 defines the maximum expanded dimension D2. In examples, fixation member 122 defines a distal end 121 ("fixation member distal end 121") configured to extend distal to balloon 124 when balloon 124 defines the maximum expanded dimension D2. For example, fixation member 122 may be configured such that fixation member distal end 121 extends distal to balloon 124 (e.g., by a displacement B2 (FIG. 4)) when balloon 124 defines the maximum expanded dimension D2. In other examples(e.g., as illustrated in FIGS. 9, 10, 13, 15, 17), balloon 124 is configured to extend distal to fixation member distal end 121 (e.g., by a displacement B4 (FIG. 9)) when balloon 124 defines the maximum expanded dimension D2. Fixation member distal end 121 may be a distal-most portion of fixation member 122 when lead body 110 (e.g., lead body distal portion 112) supports fixation member 122. Displacement B2 and or displacement B4 may be substantially parallel to longitudinal axis L.

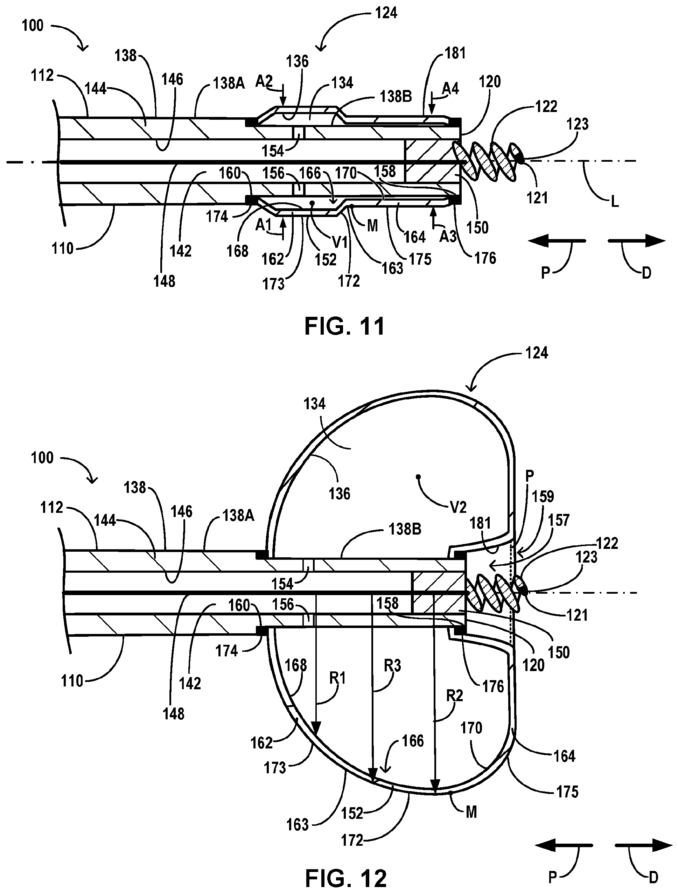

Balloon 124 (e.g., balloon body 152 (FIGS. 11-12)) defines a proximal balloon portion 162 and a distal balloon portion 164. Distal balloon portion 164 is distal to proximal balloon portion 162. Balloon 124 is configured such that distal balloon portion 164 extends distal to (e.g., beyond) lead distal end 120 when balloon 124 is in the inflated configuration (e.g., when balloon 124 substantially establishes the maximum expanded dimension D1). Balloon 124 may be configured such that distal balloon portion 164 defines maximum expanded dimension D2. Balloon 124 may be configured such that proximal balloon portion 162 gradually tapers radially outward from lead body distal portion 112 as proximal balloon portion 162 extends axially (e.g., distally) toward distal balloon portion 164 (e.g., as substantially depicted in FIGS. 4-6). In examples, balloon 124 is configured such that a dimension of proximal balloon portion 162 (e.g., a dimension substantially parallel to maximum dimension D2) gradually increases toward the maximum expanded dimension D2 defined by distal balloon portion 164. Balloon 124 may define a substantially toroidal shape surrounding lead body distal portion 112 when distal balloon portion 164 defines the maximum expanded dimension D2 and proximal balloon portion 162 gradually tapers radially outward from lead body distal portion 112 while extending axially toward distal balloon portion 164.

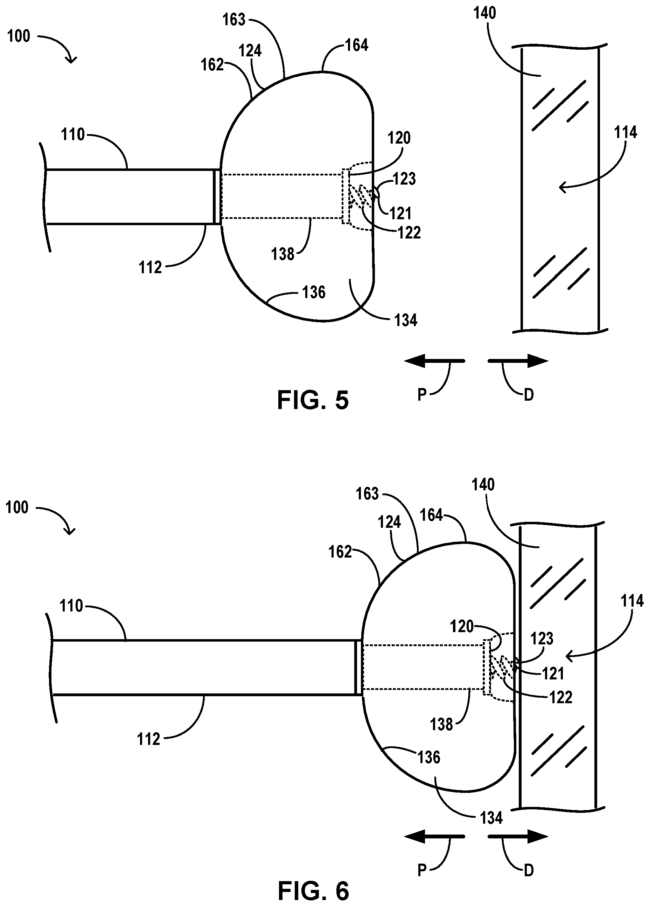

FIG. 5 illustrates medical system 100 with balloon 124 in the inflated configuration approaching a tissue wall 140. Tissue wall 140 may be, for example, a septal wall within heart 102 (FIG. 1). Balloon 124 substantially surrounds longitudinal axis L and extends distal to lead distal end 120, such that balloon 124 forms a bumper around fixation member 122 to minimize and/or eliminate physical interference between fixation member 122 and other anatomical structures within patient 104 enroute to tissue wall 140. FIG. 6 illustrates medical system 100 with balloon 124 in the inflated configuration and having positioned electrode 123 in sufficient proximity to electrically communicate with tissue wall 140. Medical system 100 may be configured such that electrode 123 electrically communicates with tissue wall 140 by substantially contacting tissue wall 140 and/or electrically communicating with tissue wall 140 in a substantially contactless manner (e.g., using the conductivity of an intervening blood volume). Electrode 123 may be electrically connected to one or more conductors (not shown) extending through lead body 110 and electrically connected to processing circuitry 125 of medical device 108 (FIG. 1). With electrode 123 in sufficient proximity to tissue wall 140, a clinician may navigate lead body distal portion 112 to potential implantation sites and evaluate the adequacy of each potential site through observation of a signal relayed from electrode 123 to processing circuitry 125. Hence, as illustrated in FIG. 6, a clinician may perform cardiac mapping using electrode 123 while balloon 124 is in the inflated configuration. The cardiac mapping may occur as balloon 124 substantially shrouds fixation member 122 to reduce and/or eliminate physical interferences between fixation member 122 and surrounding anatomical structures within patient 104. When the clinician determines medical system 100 has located an adequate target site, the clinician may deflate balloon 124 to allow insertion of fixation member 122 at the target site.

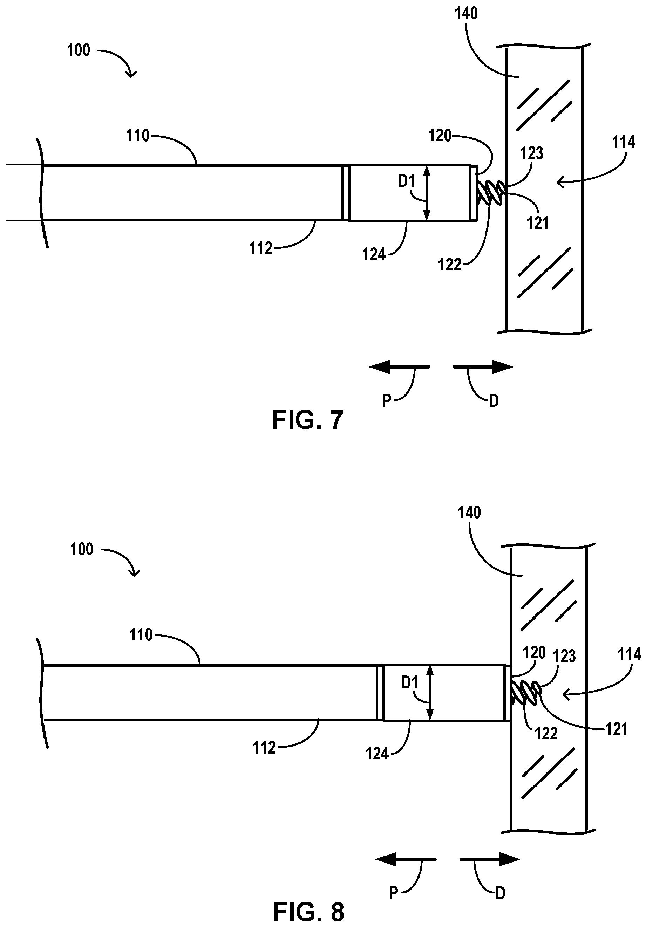

FIG 7 illustrates medical system 100 with fixation member 122 positioned to implant electrode 123 within tissues of tissue wall 140. Balloon 124 is in the deflated configuration substantially re-establishing the maximum initial dimension D1. In the deflated configuration, balloon 124 exposes a portion of or substantially all of fixation member 122 distal to lead distal end 120, such that the clinician may cause fixation member 122 to fixate to tissue wall 140. For example, when fixation member 122 defines a helix, the clinician may cause fixation member 122 to imbed into tissue wall 140 by rotating lead body 110 around longitudinal axis L. FIG. 8 illustrates medical system 100 with balloon 124 in the deflated configuration and fixation member 122 imbedded within tissue wall 140. Fixation member 122 may position electrode 123 such that circuitry 125 of medical device 108 (FIG. 1) may sense cardiac activity and/or deliver therapy to patient 104 via electrode 123.

In some examples, as depicted in FIG. 9 and FIG. 10, balloon 124 may be configured such that both proximal balloon portion 164 and distal balloon portion 164 substantially define maximum expanded dimension D2 when balloon 124 is in the inflated configuration. Proximal balloon portion 162 may expand radially outward to substantially define maximum expanded dimension D2 when balloon 124 is in the inflated configuration. In examples, proximal balloon portion 162 expands radially outward such that balloon 124 defines maximum expanded dimension D2 over some length of balloon 124 extending from proximal balloon portion 162 to distal balloon portion 164. In examples, balloon 124 is configured such that a medial portion 163 of balloon 124 ("medial balloon portion 163") substantially extending from proximal balloon portion 162 to distal balloon portion 164 defines maximum expanded dimension D2 when balloon 124 is in the inflated configuration. Balloon 124 may define a substantially toroidal shape surrounding lead body distal portion 112 when at least medial balloon portion 163 defines the maximum expanded dimension D2. FIG. 9 illustrates medical system 100 with balloon 124 in the inflated configuration approaching tissue wall 140. FIG. 10 illustrates medical system 100 with balloon 124 in the inflated configuration and having positioned electrode 123 in sufficient proximity to electrically communicate with tissue wall 140.

As discussed, medical system 100 may be configured such that electrode 123 electrically communicates with tissue wall 140 in a substantially contactless manner (e.g., using the conductivity of an intervening blood volume) when balloon 124 defined maximum expanded dimension D2. In examples, balloon 124 (e.g., distal balloon portion 164) is configured to extend distal to (or substantially even with) fixation member distal end 121 when balloon 124 defines the maximum expanded dimension D2 (e.g., when balloon 124 is in the inflated configuration). For example, balloon 124 and/or fixation member 122 may be configured such that distal balloon portion 164 extends distal to fixation member distal end 121 by a displacement B4 when balloon 124 defines the maximum expanded dimension D2 (e.g., such that fixation member distal end 121 is proximal to distal balloon portion 164). Displacement B4 may be substantially parallel to longitudinal axis L.

Medical system 100 may be configured such fixation member distal end 121 is displaced from tissue wall 140 by a displacement similar to displacement B4 when medical system 100 (e.g., distal balloon portion 164) contacts tissue wall 140. In examples, balloon 124 (e.g., distal balloon portion 164) is configured to substantially establish the displacement similar to displacement B4 when balloon 124 contacts tissue wall 140. Medical system 100 may be configured such that electrode 123 is in sufficient proximity to tissue wall 140 to electrically communicate with tissue wall 140 in a substantially contactless manner (e.g., using the conductivity of an intervening blood volume) when balloon 124 (e.g., distal balloon portion 164) substantially establishes the displacement similar to displacement B4. With electrode 123 in sufficient proximity to tissue wall 140 (e.g., with a displacement similar to the displacement B4 between fixation member 122 and tissue wall 140), a clinician may navigate lead body distal portion 112 to potential implantation sites and evaluate the adequacy of each potential site through observation of a signal relayed from electrode 123 to processing circuitry 125. The clinician may perform cardiac mapping using electrode 123 while balloon 124 substantially establishes the displacement similar to displacement B4.

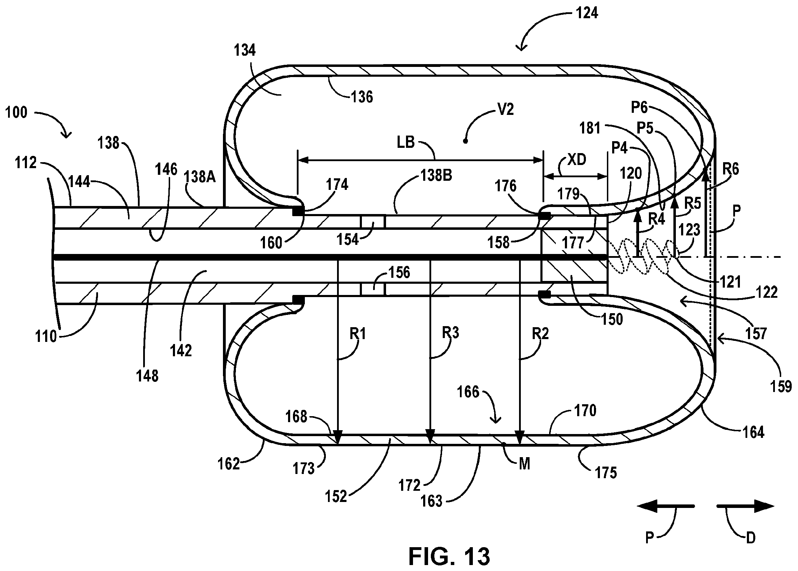

FIG. 11 illustrates a cross-sectional view of medical system 100 with balloon 124 in the deflated configuration. FIG. 12 illustrates a cross-sectional view of medical system 100 with balloon 124 in the inflated configuration. In FIG. 12, a dimension of proximal balloon portion 162 (e.g., a dimension substantially parallel to maximum dimension D2) gradually increases toward the maximum expanded dimension D2 defined by distal balloon portion 164. Fixation member 122 extends distal to balloon 124 in FIG. 12. FIG. 13 illustrates another cross-sectional view of medical system 100 with balloon 124 in the inflated configuration. In FIG. 13, medial balloon portion 163 extending from proximal balloon portion 162 to distal balloon portion 164 defines maximum expanded dimension D2. Balloon 124 extends distal to fixation member 122 in FIG. 13. The cross-sections of FIG. 11, FIG. 12, and FIG. 13 are taken with a cutting plane parallel to the page and longitudinal axis L.

Lead body distal portion 112 may define an inner lumen 142 extending at least partially through lead body distal portion 112. In examples, longitudinal axis L extends through inner lumen 142 and intersects lead distal end 120. Lead body 110 (e.g., lead body distal portion 112) may include a wall 144 defining an inner surface 146, with inner surface 146 defining inner lumen 142. Wall 144 may further define distal exterior surface 138 opposite inner surface 146. In some examples, inner lumen 142 extends to a proximal opening 139 (FIG. 1) defined in a proximal portion of lead body 110. Lead body 110 may be configured such that proximal opening 139 is extracorporeal to the patient when lead body 110 extends through vasculature of patient 104 (FIG. 1). In examples, lead body 110 is configured such that proximal opening 139 is extracorporeal to the patient when lead body distal portion 112 is positioned within a chamber of heart 102 (FIG. 1).

Medical system 100 may include a conductor 148 in electrical communication with electrode 123. In examples, fixation member 122 defines electrode 123, and conductor 148 electrically communicates with electrode 123 via fixation member 122. For example, fixation member 122 may comprise a conductive material covered by an insulative layer, and electrode 123 may be a portion of the conductive material where the insulative layer is removed. Conductor 148 may extend through inner lumen 142, although this is not required. In examples, the conductor 148 is configured to electrically communicate with processing circuitry 125 (FIG. 1) to deliver therapy to patient 104 using electrode 123. In examples, conductor 148 is configured to extend to a position extracorporeal to the patient when lead body 110 extends through vasculature of patient 104 (FIG. 1), and/or when lead body distal portion 112 is positioned within a chamber of heart 102 (FIG. 1).

In examples, medical system 100 is configured such that inner lumen 142 is fluidly isolated from patient 104 when lead body 110 extends through vasculature of patient 104 (FIG. 1). Medical system 100 may be configured such that inner lumen 142 is fluidly isolated from lead distal end 120 when lead distal end 120 is within vasculature of patient 104. In examples, medical system 100 includes a stop 150 configured to fluidly isolate inner lumen 142 and lead distal end 120 when lead distal end 120 is within vasculature of patient 104. In examples, stop 150 is configured to mechanically support fixation member 122. Hence, inner lumen 142 may be configured to such that inner lumen 142 is fluidly coupled to a proximal opening (e.g., proximal opening 139 (FIG. 1)) extracorporeal to patient 104 and fluidly isolated from lead distal end 120 when lead distal end 120 is within vasculature of patient 104.

Although depicted in FIG. 11, FIG. 12, and FIG. 13 as mechanically supported by fixation member 122, in some examples, fixation member 122 is configured to translate (e.g., distally and/or proximally) relative to stop 150 and/or inner surface 146. Fixation member 122 may be configured to translate relative to stop 150 and/or inner surface 146 when balloon 124 is in the deflated configuration and/or the inflated configuration. In examples, fixation member 122 is configured to translate relative to stop 150 and/or inner surface 146 at least from a position proximal to balloon 124 when balloon 124 is in the inflated configuration to a position distal to balloon 124 when balloon 124 is in the inflated configuration. Fixation member 122 may be configured to translate relative to stop 150 and/or inner surface 146 from a position proximal to lead body distal end 120 to a position distal to lead body distal end 120. In some examples, fixation member 122 is configured to rotate around longitudinal axis L relative to stop 150 and/or inner surface 146.

Balloon 124 may include a balloon body 152 configured to elastically expand when balloon 124 transitions from the deflated configuration to the inflated configuration (e.g., when an inflating medium is provided to interior volume 134). Balloon body may define balloon inner surface 136. In examples, balloon body 152 extends at least partially around a perimeter defined by distal exterior surface 138 (e.g., a perimeter perpendicular to longitudinal axis L). In examples, balloon body 152 extends substantially completely around the perimeter defined by distal exterior surface 138. Balloon 124 (e.g., balloon body 152) may define an outer surface 172 ("balloon outer surface 172") configured to face in a direction away from longitudinal axis L. In examples, balloon body 152 defines balloon outer surface 172 and balloon inner surface 136, with balloon outer surface 172 opposite balloon inner surface 136. Balloon outer surface 172 may include a proximal portion 173 ("proximal outer surface 173") defining at least some portion of proximal balloon portion 162 and/or a distal portion 175 ("distal outer surface 175") defining at least some portion of distal balloon portion 164.

Balloon 124 may be configured such that proximal outer surface 173 defines a first outer radius R1 extending perpendicularly from longitudinal axis L and distal outer surface 175 defines a second outer radius R2 extending perpendicularly from longitudinal axis L. In some examples, as depicted in FIG. 12, first outer radius R1 may be less than second outer radius R2. In some examples, as depicted in FIG. 13, first outer radius R1 may be substantially equal to second outer radius R2. For example, first outer radius R1 may be substantially equal to second outer radius R2 when medial balloon portion 163 defines an outer radius R3 substantially equal to or greater than first outer radius R1 and second outer radius R2.

Balloon body 152 (e.g., balloon inner surface 136) and a portion of distal exterior surface 138 may define interior volume 134. In examples, lead body distal portion 112 defines distal exterior surface 138 to include a first exterior surface 138A and a second exterior surface 138B, with first exterior surface 138A proximal to second exterior surface 138B. Balloon body 152 and second exterior surface 138B may define at least a portion of or in some cases substantially all of an inner boundary of interior volume 134. In examples, second exterior surface 138B extends substantially from first exterior surface 138A to lead distal end 120.

Medical system 100 includes one or more structures defining a lumen configured to deliver an inflating medium (e.g., air, saline, or another inflating medium) to interior volume 134 to cause inflation of balloon 124. In examples, lead body 110 defines the lumen configured to deliver the inflating medium. In some examples, inner lumen 142 is configured to deliver the inflating medium. Lead distal portion 112 may include one or more side lumens such as side lumen 154 and side lumen 156 configured to fluidly couple inner lumen 142 and interior volume 134, such that an inflating medium provided to inner lumen 142 may flow to interior volume 134 via side lumens 154, 156.

Medical system 100 may include on or more fixation structures configured to affix balloon body 152 to distal exterior surface 138. In examples, medical system 100 includes a first fixation structure 174 configured to affix balloon body 152 to distal exterior surface 138 substantially at first location 160. Medical system 100 may include a second fixation structure 176 configured to affix balloon body 152 to distal exterior surface 138 substantially at second location 158. As used here, when a portion of balloon body 152 is affixed to a portion of distal exterior surface 138, this may mean medical system 100 is configured such that the portion of balloon body 152 remains substantially stationary relative to the portion of distal exterior surface 138 as balloon 124 transitions between the deflated state and the inflated state. In some examples, medical system 100 may be configured to affix the portion of balloon body 152 using an adhesive, soldering, welding, heat shrinking, or some other joining method.

Fixation structures 174, 176 may define any shape sufficient to affix balloon body 152 to distal exterior surface 138, and may affix balloon body 152 to distal exterior surface 138 in any manner. In examples, first fixation structure 174 and/or second fixation structure 176 is configured to pin a portion of balloon body 152 between distal exterior surface 138 and the respective fixation structure. In some examples, a portion of balloon body 152 extends around a periphery of distal exterior surface 138 (e.g., a periphery surrounding some portion or substantially all of longitudinal axis L), and first fixation structure 174 or second fixation structure 176 is configured to pin the portion of balloon body 152 around the periphery. In some examples, first fixation structure 174 and/or second fixation structure 176 defines a ring structure configured to surround a portion of distal exterior surface 138 and longitudinal axis L. In some examples, first fixation structure 174 and/or second fixation structure 176 may be a cord or suture wound around lead body distal portion 112 (e.g., surrounding longitudinal axis L), an adhesive affixing balloon body 152 to distal exterior surface 138, or another structure and/or component of medical system 100 configured to affix balloon body 152 to distal exterior surface 138.

As discussed, balloon 124 is configured to expand radially outward from distal exterior surface 138 (e.g., second exterior surface 138B) and extend distally beyond lead distal end 120 when balloon 124 inflates from the deflated configuration (FIG. 11) to the inflated configuration (FIG. 12, FIG. 13). In examples, balloon body 152 is affixed to distal exterior surface 138 at least at a location 158 (e.g., by second fixation structure 176), and balloon body 152 is configured to extend distal to location 158 when balloon 124 is in the inflated configuration. In some examples, balloon body 152 is affixed to exterior surface 138 at first location 160 (e.g., by first fixation structure 174) and a second location such as location 158, with first location 160 proximal to second location 158. Balloon body 152 may define a midpoint M between (e.g., substantially halfway between) first location 160 and second location 158 and separating proximal balloon portion 162 and distal balloon portion 164. Proximal balloon portion 162 may extend between first location 160 and midpoint M, and distal balloon portion 164 may extend between midpoint M and second location 158. Balloon 124 may be configured such that distal balloon portion 164 is distal to second location 158 when balloon 124 is in the inflated configuration. In examples, balloon 124 (e.g., balloon body 152) is configured to be proximal to lead distal end 120 when balloon 124 is in the deflated configuration. Balloon 124 (e.g., balloon body 152) may be configured to expand in a direction perpendicular to longitudinal axis L when balloon 124 expands radially outward from distal exterior surface 138.

Balloon 124 may be configured to expand asymmetrically to cause balloon 124 (e.g., distal balloon portion 164) to extend distal to lead distal end 120 when balloon 124 inflates to the inflated configuration. For example, balloon 124 may be configured such that proximal balloon portion 162 is distal to first location 160 and distal balloon portion 164 is distal to second location 158 when balloon 124 is in the inflated configuration. In some examples, balloon 124 is configured to cause a center of volume of interior volume 134 to shift in the distal direction D when balloon 124 inflates. For example, balloon 124 may be configured such that interior volume 134 defines a first center of volume V1 in the deflated configuration (FIG. 11) and a second center of volume V2 in the inflated configuration (FIG. 12, FIG. 13). Balloon 124 may be configured such that the second center of volume V2 is distal to the first center of volume V1. In some examples, balloon 124 is configured to extend distal to lead distal end 120 and substantially surround some portion of fixation member 122 to define a distal cavity 157 around fixation member 122. For example, distal cavity 157 may at least partially surround longitudinal axis L and the portion of fixation member 122. Balloon 124 may define an opening 159 to distal cavity 157 ("cavity opening 159") such that a fluid (e.g., blood) may substantially occupy distal cavity 157 when balloon 124 is in the inflated configuration and lead distal end 120 is immersed in the fluid. Medical system 100 may be configured such that electrode 123 may electrically with tissue (e.g., tissue wall 140) in a substantially contactless manner using the conductivity of an intervening blood volume within distal cavity 157.

Balloon 124 may be configured such that balloon outer surface 172 (e.g., distal outer surface 175) defines at least a portion of distal cavity 157 and/or cavity opening 159 when balloon 124 is in the inflated configuration. In examples, distal outer surface 175 includes a cavity outer surface 181 configured to define at least the portion of distal cavity 157 and/or cavity opening 159 when balloon 124 is in the inflated configuration. Distal cavity 157 may be, for example, a recess bounded at least in part by cavity outer surface 181 and lead distal end 120. Balloon 124 may be configured to define distal cavity 157 to substantially surround a portion of longitudinal axis L distal to lead distal end 120 when balloon 124 is in the inflated configuration. In some examples, distal cavity 157 is a volume defined at least in part by cavity outer surface 181, cavity opening 159, and lead distal end 120.

In examples, cavity outer surface 181 is configured to substantially face away from longitudinal axis L when balloon 124 is in the deflated configuration (e.g., FIG. 11). Cavity outer surface 181 may be configured to substantially faces toward longitudinal axis L when balloon 124 is in the inflated condition (e.g., FIG. 12, FIG. 13). In examples, cavity outer surface 181 is configured to transition from an orientation facing away from longitudinal axis L to an orientation facing toward longitudinal axis L when distal balloon portion 164 extends distal to lead distal end 120 (e.g., when balloon 124 inflates from the deflated configuration to the inflated configuration). Cavity outer surface 181 may be configured to substantially surround a portion of longitudinal axis L distal to lead distal end 120 when cavity outer surface 181 transitions to the orientation facing toward longitudinal axis L.

Cavity opening 159 is an opening defined by distal outer surface 175 when balloon 124 inflates to cause distal balloon portion 164 to extend distal to lead distal end 120. In examples, distal outer surface 175 defines a perimeter P defining a boundary that substantially surrounds cavity opening 159. In examples, cavity outer surface 181 defines perimeter P. Perimeter P is illustrated in dashed line and perpendicular to the page in FIG. 13. In examples, perimeter P is a closed boundary surrounding cavity opening 159 and longitudinal axis L. In some examples, distal outer surface 175 defines perimeter P around a distal-most portion of distal balloon portion 164 when distal balloon portion 164 to extend distal to lead distal end 120 (perimeter P is illustrated proximal to the distal-most portion in FIG. 13 for clarity). In some examples, perimeter P is substantially perpendicular to longitudinal axis L.

Fixation member 122 is configured to extend longitudinally with distal cavity 157. Distal cavity 157 may be a substantially bell-shaped cavity with cavity opening 159 defining the mouth of the bell shape and/or cavity outer surface 181 defining some portion of the waist of the bell shape. In examples, balloon 124 is configured such that balloon outer surface 172 (e.g., cavity outer surface 181) substantially flares radially away from fixation member 122 when balloon 124 is in the inflated configuration. Distal outer surface 175 and/or cavity outer surface 181 may flare radially away from fixation member 122 to increase a displacement from distal outer surface 175 and fixation member 122 as fixation member 122 extends distally from lead body distal portion 112. The radial flare of distal outer surface 175 and/or cavity outer surface 181 may provide the increasing displacement from fixation member 122 to, for example, minimize and/or substantially reduce a likelihood of contact between balloon body 152 and fixation member 122 when medical system 100 is within vasculature and/or in proximity to tissue wall 140 within a patient. For example, the radial flare of distal outer surface 175 and/or cavity outer surface 181 may minimize and/or substantially reduce contact between balloon body 152 and fixation distal end which might lead to a puncture of balloon 124 by fixation member distal end 121 or another portion of fixation member 122.

In examples, balloon 124 is configured to increase a radial displacement between longitudinal axis L and distal outer surface 175 and/or cavity outer surface 181 as fixation member 122 extends from lead body distal portion 112 in a direction substantially along longitudinal axis L. For example, FIG. 13 illustrates balloon 124 forming distal cavity 157 such that distal outer surface 175 and/or cavity outer surface 181 defines at least a fourth radial displacement R4 from longitudinal axis L, a fifth radial displacement R5 from longitudinal axis L, and a sixth radial displacement R6 from longitudinal axis L. Fifth radial displacement R5 may be greater than fourth radial displacement R4 and/or sixth radial displacement R6 may be greater than fifth radial displacement R5, such that balloon 124 increases the radial displacement from longitudinal axis L to distal outer surface 175 and/or cavity outer surface 181 as a distance from lead distal end 120 increases.

Balloon 124 may define fourth radial displacement R4 from longitudinal axis L to a point P4 on distal outer surface 175 and/or cavity outer surface 181, define fifth radial displacement R5 from longitudinal axis L to a point P5 on distal outer surface 175 and/or cavity outer surface 181, and/or define sixth radial displacement R6 from longitudinal axis L to a point P6 on distal outer surface 175 and/or cavity outer surface 181. In examples, P4 is proximal to point P5, and point P5 is proximal to point P6, such that distal outer surface 175 and/or cavity outer surface 181 substantially flares radially outward away from fixation member 122. In examples, fourth radial displacement R4, fifth radial displacement R5, and/or sixth radial displacement R6 may be substantially perpendicular to longitudinal axis L.

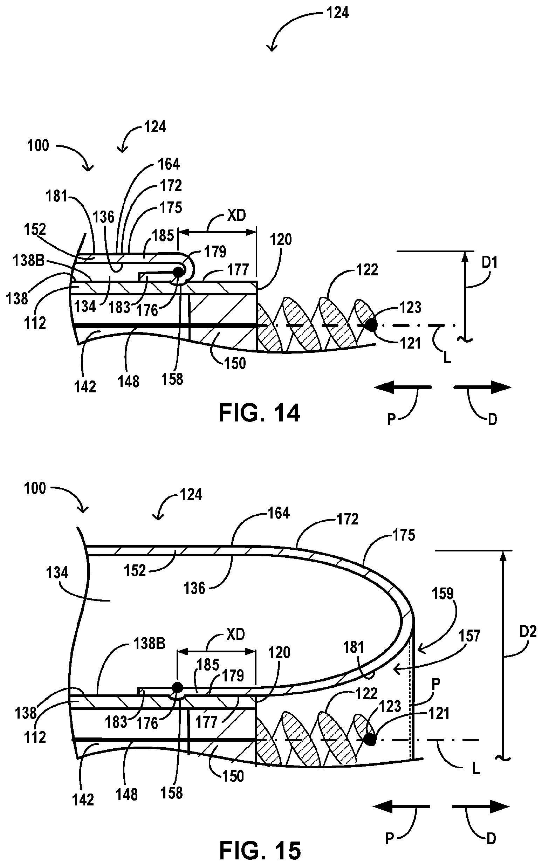

In examples, distal exterior surface 138 (e.g., second exterior surface 138B) is configured to support at least some portion of distal balloon portion 164 to cause and/or assist outer surface 173 and/or cavity outer surface 181 in radially flaring away from fixation member 122. For example, second location 158 and/or second fixation structure 176 may be displaced proximally from lead distal end 120 such that second exterior surface 138B provides a supporting surface to distal balloon portion 164 when distal balloon portion 164 inflates to extend distal to second location 158 and/or second fixation structure 176. For example, FIG. 14 is a cross-sectional schematic illustration of a portion of medical system 100 including distal balloon portion 164 and a portion of lead body distal portion 112 including lead distal end 120. Medical system 100 is in a deflated configuration defining maximum initial dimension D1 in FIG. 14. Second location 158 and/or second fixation structure 176 are displaced proximally from lead distal end 120 by a displacement XD. In examples, displacement XD is defined by a portion of second exterior surface 138B. In some examples, displacement XD is substantially parallel to longitudinal axis L.

Second location 158 and/or second fixation structure 176 may be displaced from lead distal end 120 by the displacement XD such that some portion of distal exterior surface 138 (e.g., second exterior surface 138B) defines a supporting surface 177. Supporting surface 177 may be configured to support distal balloon portion 164 to, for example, cause and/or assist outer surface 173 and/or cavity outer surface 181 to radially flare in a direction away from fixation member 122 when distal balloon portion 164 extends distal to second location 158 and/or second fixation structure 176. Supporting surface 177 may be a portion of distal exterior surface 138. In examples, supporting surface 177 is configured to contact at least some portion of distal outer surface 175 when distal balloon portion 164 extends distal to second location 158 and/or second fixation structure 176. In examples, distal outer surface 175 defines a contact outer surface 179 configured to contact supporting surface 177 when distal balloon portion 164 extends distal to second location 158 and/or second fixation structure 176. Supporting surface 177 may be configured to substantially constrain and/or cease the expansion of at least some portion of distal balloon portion 164 (e.g., outer surface 179) toward fixation element 122. The constraint and/or cessation of the expansion of the portion of distal balloon portion 164 may cause and/or assist outer surface 173 and/or cavity outer surface 181 in radially flaring in a direction away from fixation member 122.

For example, FIG. 15 is a cross-sectional schematic illustration of the portion of medical system 100 with medical system 100 in an inflated configuration, such that distal balloon portion 164 extends distal to second location 158 and/or second fixation structure 176. Medical system 100 defines maximum expanded dimension D2 in FIG. 15. Distal balloon portion 164 is extended distal to second location 158 and/or second fixation structure 176 such that supporting surface 177 supports (e.g., contacts) outer surface 179. In examples, supporting surface 177 substantially constrains and/or ceases the expansion of at least outer surface 179 toward fixation element 122 when supporting surface 177 supports outer surface 179. The support provided to outer surface 179 by supporting surface 177 may cause and/or assist distal outer surface 175 in radially flaring away from fixation member 122 when distal outer surface 175 defines distal cavity 157 and/or cavity opening 159. In examples, outer surface 179 is configured to may cause and/or assist distal outer surface 175 to define fourth radial displacement R4, fifth radial displacement R5, and/or sixth radial displacement R6 (FIG. 13).

Medical system 100 may displace second location 158 and/or second fixation structure 176 from proximally from lead distal end 120 by any amount. In examples, medical system 100 defines a length LB (FIG. 13) from first location 160 to second location 158. Length LD may be substantially parallel to longitudinal axis L. In examples, displacement XD is at least 5% of the length of length LB, at least 10% of the length of length LB, at least 20% of the length of length LB, or another percentage of the length of length LB. In some examples, outer surface 179 and cavity outer surface 181 join to form a substantially continuous portion of distal outer surface 175. For example, outer surface 179 may define a first portion of distal outer surface 175 and cavity outer surface 181 may define a second portion of distal outer surface 175 joined to the first portion. In examples, cavity outer surface 181 is proximal to outer surface 179 when cavity outer surface 181 faces away from longitudinal axis L (e.g., when balloon 124 is in the deflated configuration) and distal to outer surface 179 when cavity outer surface 181 faces toward longitudinal axis L (e.g., when balloon 124 is in the inflated condition).

In some examples, instead of or in addition to contacting supporting surface 177, distal balloon portion 164 substantially folds over itself in the vicinity of second location 158 to cause and/or assist outer surface 173 and/or cavity outer surface 181 in defining a substantially bell-shaped distal cavity 157. Distal balloon portion 164 may fold over itself such that, when medical system 100 is in the deflated configuration (e.g., FIG. 14), a first portion of distal balloon portion 164 substantially lies between a second portion of distal balloon portion 164 and distal exterior surface 138. The folding of distal balloon portion 164 in the vicinity of second location 158 may cause and/or assist outer surface 173 and/or cavity outer surface 181 to define the substantially bell-shaped distal cavity 157 when balloon 124 inflates and medical system 100 is in the inflated configuration (e.g., FIG. 15).

As illustrated in FIG. 14 and FIG. 15, distal balloon portion 164 includes a first portion 183 ("distal first portion 183") and a second portion 185 ("distal second portion 185"). Distal first portion 183 may define at least a portion of contact outer surface 179. Distal balloon portion 164 is substantially folded such that distal first portion 183 lies between distal second portion 185 and distal exterior surface 138 when distal balloon portion 164 is attached to distal exterior surface 138 and medical system 100 is in a deflated configuration. In examples, distal balloon portion 164 substantially folds around second fixation structure 176. Distal balloon portion 164 may substantially fold around second fixation structure 176 such that balloon body 152 extends distal to second fixation structure 176 and substantially wraps around second fixation structure 176 to position distal first portion 183 between distal second portion 185 and distal exterior surface 138. In examples, second fixation structure 176 is positioned substantially between distal first portion 183 and distal second portion 185 when distal balloon portion 164 substantially wraps around second fixation structure 176. In examples, second fixation structure 176 substantially pins at least a portion of distal first portion 183 to second location 158. The folding of distal balloon portion 164 in the vicinity of second location 158 may cause and/or assist outer surface 173 and/or cavity outer surface 181 to define the substantially bell-shaped distal cavity 157 when balloon 124 inflates.

In examples, second fixation structure 176 substantially pins at least a portion of distal first portion 183 to second location 158. Second fixation structure 176 may substantially pin distal first portion 183 to second location 158 such that second portion 185 extends distal to distal first portion 183 when balloon 124 inflates and medical system 100 transitions to the inflated configuration (FIG. 15). In examples, second portion 185 extends distal to distal first portion 183 and second fixation structure 176 when balloon 124 inflates and medical system 100 transitions to the inflated configuration. In examples, second fixation structure 176 may substantially pin distal first portion 183 to second location 158 to cause cavity outer surface 181 to transition from the orientation facing away from longitudinal axis L to the orientation facing toward longitudinal axis L when medical system 100 transitions from the deflated state to the inflated state.

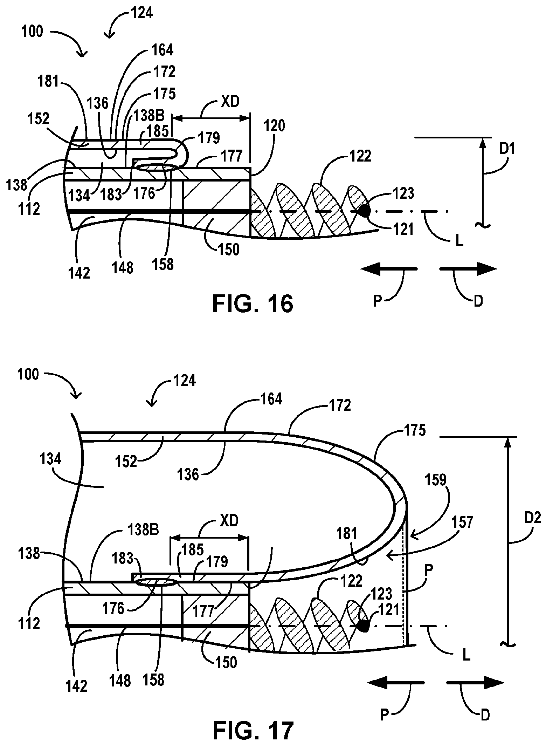

In some examples, medical system 100 is configured such that second fixation structure 176 is positioned between distal first portion 183 and distal exterior structure 138 when distal balloon portion 164 substantially folds over itself in the vicinity of second location 158. For example, FIG. 16 is a cross-sectional schematic illustration of a portion of medical system 100 including distal balloon portion 164 and a portion of lead body distal portion 112 including lead distal end 120, with medical system 100 is in a deflated configuration defining maximum initial dimension D1. FIG. 17 is a cross-sectional schematic illustration of a portion of medical system 100 including distal balloon portion 164 and a portion of lead body distal portion 112 including lead distal end 120, with medical system 100 is in an inflated configuration defining maximum initial dimension D2. Distal balloon portion 164 is configured such that distal first portion 183 positions between distal second portion 185 and distal exterior structure 138 when second fixation structure 176 is positioned between distal first portion 183 and distal exterior structure 138. Second fixation structure 176, when positioned between distal first portion 183 and distal exterior structure 138, may cause cavity outer surface 181 to transition from the orientation facing away from longitudinal axis L to the orientation facing toward longitudinal axis L when medical system 100 transitions from the deflated state to the inflated state.

In some examples, balloon 124 may be configured such that proximal balloon portion 162 and distal balloon portion 164 exhibit differing resistances to expansion when an inflating medium enters interior volume 134 (e.g., via side lumen 154, 156). For example, when the inflating medium acts against balloon inner surface 136 to cause expansion of balloon 124, proximal balloon portion 162 may resist the expansion to a differing degree than distal balloon portion 164, such that proximal balloon portion 162 and distal balloon portion 164 radially expand at least initially at different rates. In some examples, balloon 124 may be configured such that proximal balloon portion 162 has a lower resistance to expansion that distal balloon portion 164, such that proximal balloon portion 162 radially expands at least initially at a higher rate than distal balloon portion 164. The higher rate of expansion of proximal balloon portion 162 may cause distal balloon portion 164 to substantially "roll over" second location 158 as balloon 124 inflates, such that balloon 124 extends distally beyond lead distal end 120.

For example, proximal balloon portion 162 may be configured such that proximal outer surface 173 expands to define first radial displacement R1 prior to distal outer surface 172 expanding to define second radial displacement R2 as an inflating medium exerts a pressure against balloon inner surface 136. Proximal balloon portion 162 may substantially cease expansion when first radial displacement R1 is achieved as distal balloon portion 164 continues to yield enroute to achieving second radial displacement R2. The continued yield of distal portion 164 may facilitate and/or assist balloon 124 in causing distal balloon portion 164 to extend distal to lead distal end 120 as balloon 124 inflates.

In some examples, balloon 124 may be configured to cause distal balloon portion 164 to extend distally beyond lead distal end 120 by altering an interior surface area of balloon inner surface 136 ("balloon surface area 166") relative to a location on distal exterior surface 138 (e.g., second exterior surface 138B). Altering the balloon surface area 166 may, for example, cause the forces exerted by the inflating medium to differ over interior surface area as balloon 124 inflates, causing proximal balloon portion 164 and distal balloon portion 164 to expand at different rates. In examples, balloon 124 is configured to alter balloon surface area 166 as balloon body 152 extends distally from first location 160 to second location 158 in the deflated configuration. For example, balloon 124 may define a first surface area 168 (FIGS. 11-13) comprising balloon surface area 166 within proximal balloon portion 162. Balloon 124 may define a second surface area 170 (FIGS. 11-13) comprising balloon surface area 166 within distal balloon portion 164. First surface area 168 may be greater than second surface area 170, such that an inflating medium entering interior volume 134 (e.g., via side lumens 154, 156) exerts a greater force against proximal balloon portion 162 than distal balloon portion 164. The greater force on proximal balloon portion 162 may substantially cause distal balloon portion 164 to "roll" distally and extend over lead body distal end 120 as balloon 124 is inflated.

In some examples, balloon 124 is configured such that proximal outer surface 173 defines a first outer dimension (e.g., a first diameter) and distal outer surface 175 defines a second outer dimension (e.g., a second diameter), with the first outer dimension greater than the second outer dimension. For example, balloon 124 may define the first outer dimension between the arrows A1 and A2 and define the second outer dimension between the arrows A3 and A4 of FIG. 11. In examples, the first outer dimension and the second outer dimension are perpendicular to the longitudinal axis L. The first outer dimension and the second outer dimension may be configured to cause distal balloon portion 164 to roll distally and extend over lead body distal end 120 as balloon 124 is inflated. For example, the first outer dimension and the second outer dimension may be configured to cause first surface area 168 to exceed second surface area 170.

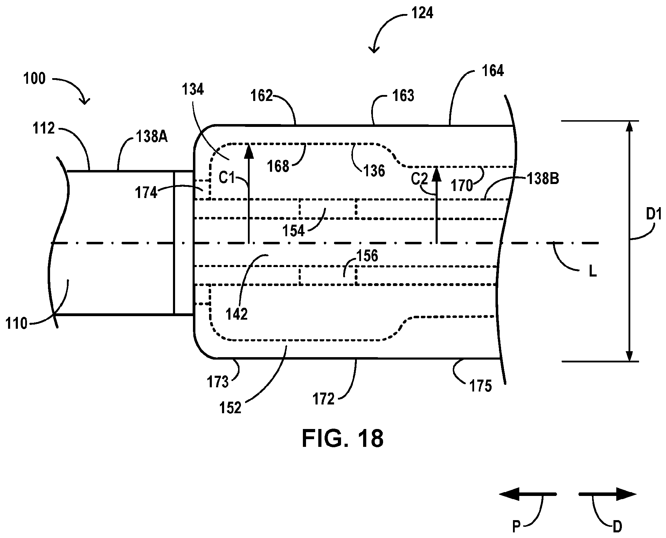

In other examples, balloon 124 may be configured such that balloon inner surface 136 defines one or more inner dimensions configured to cause distal balloon portion 164 to extend over lead body distal end 120 as balloon 124 inflates. For example, FIG. 18 is a schematic illustration of a portion of medical system 100 including proximal balloon portion 162, medial balloon portion 163, and a portion of distal balloon portion 164. Medical system 100 is in a deflated configuration defining maximum initial dimension D1 in FIG. 18. Hidden features are shown as dashed lines in FIG. 18 for clarity. Balloon inner surface 136 may be configured to alter balloon surface area 166 as balloon body 152 extends distally from proximal balloon portion 162 to distal balloon portion 164. Balloon inner surface 136 may be configured to cause first surface area 168 to differ from second surface area 170.

In examples, balloon inner surface 136 defines a first inner dimension C1 (e.g., a first inner diameter) in proximal balloon portion 162 Balloon inner surface 136 may define a second inner dimension C2 (e.g., a second inner diameter) in distal balloon portion 164. First inner dimension C1 and second inner dimension C2 may be configured to cause distal balloon portion 164 to roll distally and extend over lead body distal end 120 as balloon 124 is inflated. For example, balloon inner surface 136 may be configured to cause first inner dimension C1 to differ from second inner dimension C2 to cause first surface area 168 to differ from second surface area 170. In examples, first inner dimension C1 is greater than the second inner dimension C2 such that, for example, first surface area 168 exceeds second surface area 170. In examples, first inner dimension C1 and second inner dimension C2 define a displacement from longitudinal axis L to balloon inner surface 136. In examples, first inner dimension C1 and second inner dimension C2 are perpendicular to longitudinal axis L. In some examples, balloon outer surface 172 defines a substantially constant outer dimension (e.g., an outer diameter) as balloon inner surface defines first inner diameter C1 and second outer diameter C2.

In some examples, balloon 124 is configured such that a yield strength of proximal balloon portion 162 differs from a yield strength of distal balloon portion 164. Balloon 124 may be configured such that the yield strength of proximal balloon portion 162 differs from the yield strength of distal balloon portion 164 to, for example, cause distal balloon portion 164 to substantially "roll over" second location 158 as balloon 124 inflates, such that balloon 124 extends distally beyond lead distal end 120. In examples, the yield strength of proximal balloon portion 162 may be a stress within proximal balloon portion 162 when proximal balloon portion 162 establishes first outer radius R1 and/or first inner dimension C1. The yield strength of distal balloon portion 164 may be a stress within distal balloon portion 164 when distal balloon portion 164 establishes second outer radius R2 and/or second inner dimension C2.

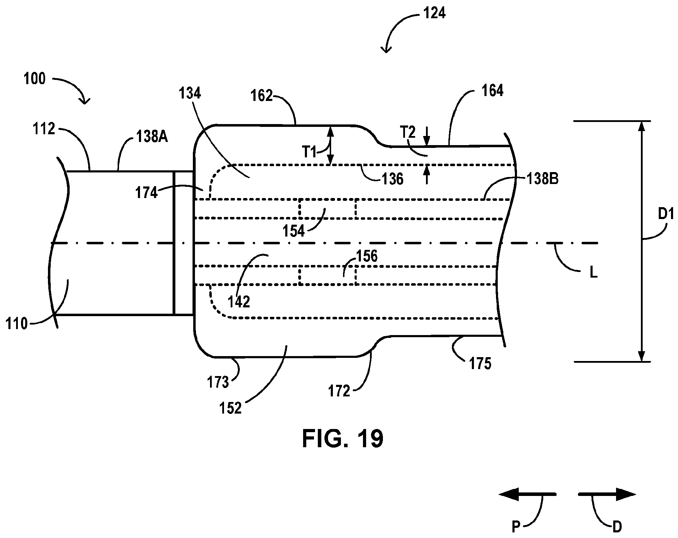

In some examples, balloon 124 may be configured to cause distal balloon portion 164 to extend distally beyond lead distal end 120 by altering a thickness of distal balloon portion 164 relative to proximal balloon portion 162. For example, FIG. 19 is a schematic illustration of a portion of medical system 100 including proximal balloon portion 162, medial balloon portion 163, and a portion of distal balloon portion 164. Medical system 100 is in a deflated configuration defining maximum initial dimension D1 in FIG. 19. Hidden features are shown as dashed lines in FIG. 19 for clarity. Balloon 124 (e.g., balloon body 152) may be configured to alter a thickness of distal balloon portion 164 relative to proximal balloon portion 162 as balloon body 152 extends distally from proximal balloon portion 162 to distal balloon portion 164. The thickness may be, for example, a distance defined by balloon body 152 between balloon inner surface 136 and balloon outer surface 172.

In examples, balloon body 152 defines a first thickness T1 in proximal balloon portion 162 Balloon inner surface 136 may define a second thickness T2 in distal balloon portion 164. First thickness T1 and second thickness T2 may be configured to cause distal balloon portion 164 to roll distally and extend over lead body distal end 120 as balloon 124 is inflated. For example, balloon body 152 may be configured to cause first thickness T1 to differ from second thickness T2 to cause distal balloon portion 164 to extend distally beyond lead distal end 120 as an inflating medium enters interior volume 134 (e.g., via side lumen 154, 156). In examples, first thickness T1 is greater than second thickness T2. In examples, first thickness T1 and second thickness T2 are substantially perpendicular to longitudinal axis L. In some examples, balloon inner surface 136 defines a substantially constant inner dimension (e.g., an inner diameter) as balloon body 152 defines first thickness T1 and second thickness T2.

In some examples, in addition to or instead of defining the first thickness T1 and the second thickness T2, balloon body 152 may include a first material and a second material configured to cause the yield strength of proximal balloon portion 162 to differ from the yield strength of distal balloon portion 164. For example, balloon body 152 may include the first material in proximal balloon portion 162. Balloon body 152 may include the second material in distal balloon portion 164. A yield strength of the first material may differ from a yield strength of the second material. In examples, the yield strength of the first material relative to the second material causes the yield strength of proximal balloon portion 162 be greater than the yield strength of distal balloon portion 164.

Although the radially flare of distal outer surface 175 in a direction away from fixation member 122 is discussed for illustration relative to FIG. 13, with medial balloon portion 163 defining maximum expanded dimension D2, distal outer surface 175 may radially flare outward when balloon 124 establishes any shape, such as when a dimension of proximal balloon portion 162 gradually increases toward the maximum expanded dimension D2 defined by distal balloon portion 164 (e.g., as depicted in FIG. 12). Likewise, distal outer surface 175 may radially flare outward when fixation member 122 extends distal to distal balloon portion 164 (as depicted in FIG. 12), when distal balloon portion 164 extends distal to fixation member 122 (as depicted in FIG. 13), and/or when distal balloon portion 164 extends distally beyond lead distal end 120 substantially the same amount as fixation member 122. Likewise, medical system 100 may include supporting surface 177, contact outer surface 179, distal first portion 183, and/or distal second portion 185, and/or may define first inner dimension C1, first inner dimension C2, first thickness T1, second thickness T2, and/or displacement XD, when balloon 124 establishes any shape, and/or when fixation member 122 extends distal to distal balloon portion 164 or distal balloon portion 164 extends distal to fixation member 122.

Balloon 124 may be configured to such that an imaging system extracorporeal to patient 104 may capture an image of balloon 124 when balloon 124 is within patient 104. In examples, balloon 124 is configured to increase an echogeneity and/or radiopacity of balloon body 152. Balloon 124 may be configured to increase its visibility for any imaging modality configured to capture images of balloon 124 when balloon 124 is within patient 104, including ultrasound, intra-vascular ultrasound (IVUS), two dimensional (2D) ultrasound, three dimensional (3D) ultrasound, four dimensional ( 4D) ultrasound, high frequency ultrasound (HIFU), isocentric fluoroscopy, bi-plane fluoroscopy, x-ray images (e.g., two dimensional x-ray images, three dimensional x-ray images, etc.), computed tomography (CT), multi-slice computed tomography (MSCT), magnetic resonance imaging (MRI), optical coherence tomography (OCT), intraoperative CT, intraoperative MRI, and/or others.

In examples, balloon body 152 is a multi-layer balloon body including a reflecting layer configured to increase a visibility (e.g., an echogeneity and/or radiopacity) of balloon body 152. For example, balloon body 152 may include an inner layer comprising balloon inner surface 136 and an outer layer comprising balloon outer surface 172, with at least one of the inner layer, the outer layer, or an additional layer between the inner layer and the outer layer configured as the reflecting layer. The reflecting layer may include particles embedded within the reflecting layer configured to increase the visibility of balloon 124. In some examples, the reflecting layer includes particles comprising tungsten (e.g., tungsten carbide), tantalum, and/or platinum. In some examples, one or more layers of balloon body 152 may exhibit a surface texture configured to increase the visibility of balloon 124. In examples, balloon 124 (e.g., balloon body 152) comprises a silicone material. In some examples, balloon 124 is a dipped balloon fabricated using a dip molding process.

In some examples, balloon 124 is configured to contact some portion of fixation member 122 when balloon 124 inflates to the inflated configuration. For example, balloon 124 may be configured to contact a portion of fixation member 122 proximal to a distal end of fixation member 122. Balloon 124 may be configured to alter an impedance of fixation member 122 when balloon 124 contacts the portion of fixation member 122. In examples, medical system 100 includes processing circuitry (e.g., within a lead analyzer) configured to sense an impedance and/or impedance alteration of fixation member 122 to provide, for example, an indication to a clinician that balloon 124 is in the inflated configuration.

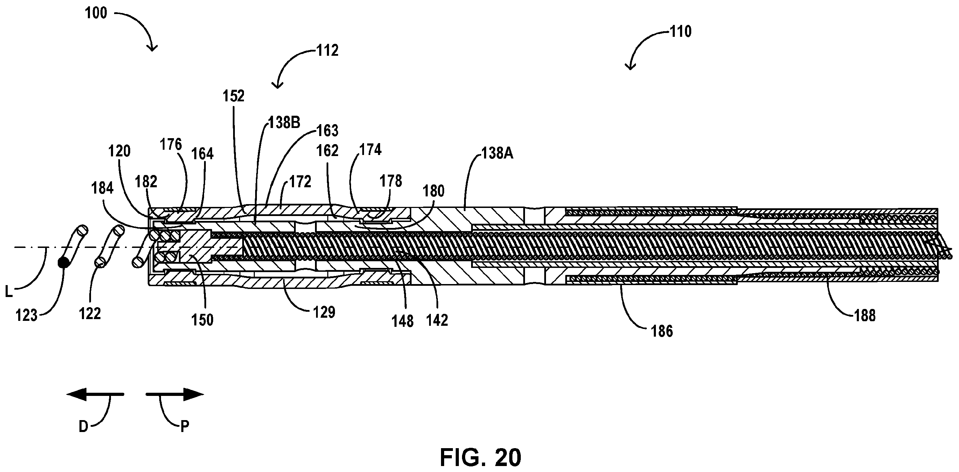

FIG. 20 illustrates a cross-sectional view of a medical system 100, taken with a cutting place perpendicular to the page and longitudinal axis L. Balloon body 152 is affixed to distal exterior surface 138 by first fixation structure 174 and second fixation structure 176. In FIG. 20, first fixation structure 174 is configured to pin a portion of proximal balloon portion 162 between second exterior surface 138B and first fixation structure 174 around a periphery of second exterior surface 138B. In examples, balloon body 152 defines a recess 178 ("balloon recess 178") configured to receive some portion of first fixation structure 174. Balloon recess 178 may be defined at least in part by a portion of balloon outer surface 172. In examples, balloon recess 178 extends around at least a portion of an outer periphery defined by balloon outer surface 172. In some examples, balloon recess 178 surrounds longitudinal axis L.

In examples, second exterior surface 138B defines a recess 180 ("surface recess 180") configured to receive a portion of proximal balloon portion 162. Surface recess 180 may be configured to receive the portion of proximal balloon portion 162 when balloon recess 178 receives first fixation structure 174. In some examples, lead body distal portion 112 is configured such that surface recess 180 receives proximal balloon portion 162 and balloon recess 178 receives first fixation structure 174 when proximal balloon portion 162 is affixed to lead body distal portion 112. In some examples, balloon recess 178 and surface recess 180 are substantially aligned (e.g., aligned relative to longitudinal axis L), such that when first fixation structure 174 exerts a force toward longitudinal axis L on a surface defining balloon recess 178, proximal balloon portion 162 inserts into surface recess 180.

In examples, balloon body 152 defines a second recess 182 ("balloon recess 182") configured to receive some portion of second fixation structure 176. Balloon recess 182 may be defined at least in part by a portion of balloon outer surface 172. In examples, balloon recess 182 extends around at least a portion of an outer periphery defined by balloon outer surface 172, and may surround longitudinal axis L. Second exterior surface 138B may define a second recess 184 ("surface recess 184") configured to receive a portion of distal balloon portion 164. Surface recess 184 may be configured to receive the portion of distal balloon portion 164 when balloon recess 182 receives second fixation structure 176. Lead body distal portion 112 may be configured such that surface recess 184 receives distal balloon portion 164 and balloon recess 182 receives second fixation structure 176 when distal balloon portion 164 is affixed to lead body distal portion 112. In examples, balloon recess 182 and surface recess 184 are substantially aligned (e.g., aligned relative to longitudinal axis L), such that when second fixation structure 176 exerts a force toward longitudinal axis L on a surface defining balloon recess 182, distal balloon portion 164 inserts into surface recess 184. Balloon body 152 may define balloon recess 182 such that balloon recess 182 is distal to balloon recess 178. Second exterior surface 138B may define surface recess 184 such that surface recess 184 is distal to surface recess 180.

Lead body 110 may be configured to flex and bend to allow, for example, navigation of medical system 100 through patient 104. In examples, lead body 110 is configured to define a curvature. Other components of medical system 100, such as fixation member 122, balloon 124, stop 150, conductor 148, and others, may be configured to retain the configurations and functionality described herein when lead body 110 defines the curvature. In examples, conductor 148 is configured to define a curvature (e.g., substantially the same curvature or a different curvature from lead body 110) when lead body 110 defines a curvature. In some examples, conductor 148 is configured to expand and/or contract lengthwise (e.g., along longitudinal axis L) when lead body 110 defines a curvature. In an example, as illustrated in FIG. 20, conductor 148 may include a helical coil extending through inner lumen 142 and surrounding longitudinal axis L.

In some examples, medical system 100 includes a second electrode 186 in electrical communication with processing circuitry 125 (FIG. 1). Second electrode 186 may be configured to act as a return electrode when electrode 123 senses cardiac activity and/or delivers therapy to patient 104. In examples, second electrode 186 is electrically connected to a second conductor 188 extending through lead body 110 and electrically connected to processing circuitry 125. Second electrode 186 and/or second conductor 188 may be mechanically supported by lead body 110. Second conductor 188 and/or second electrode 186 may be configured to define a curvature (e.g., substantially the same curvature or a different curvature from lead body 110) when lead body 110 defines a curvature. In some examples, second conductor 188 and/or second electrode 186 is configured to expand and/or contract lengthwise (e.g., along longitudinal axis L) when lead body 110 defines a curvature. In examples, as illustrated in FIG. 20, second conductor 188 and/or second electrode 186 includes a helical coil surrounding inner lumen 142 and longitudinal axis L. In some examples, second conductor 188 includes a conductive material covered by an insulative layer, and second electrode 186 is defined by a portion of the conductive material where the insulative layer is removed.

In examples, balloon 124 is a first balloon and medical system 100 includes a second balloon, such as second balloon 190 of FIG. 12. Second balloon 190 may be affixed to exterior surface 138 of lead body 110 and define a deflated figuration and an inflated configuration in a manner similar to that described for balloon 124. Second balloon 190 may be configured similarly to or in the same manner as balloon 124. For example, second balloon 190 may include a balloon body 192 defining an interior volume 194 which expands when second balloon 190 receives an inflating medium (e.g., via side lumen 156). Second balloon 190 may be configured to expand radially outward from exterior surface 138 and extend distally beyond lead distal end 120 when second balloon 190 inflates from the deflated configuration to the inflated configuration. In examples, fixation member 122 may be configured to extend distal to second balloon 190 when second balloon 190 is in the inflated configuration. In examples, second balloon 190 may be configured to extend distal to, or substantially even with, fixation member 122 when second balloon 190 is in the inflated configuration. Medical system 100 may include any number of balloons configured in like manner to balloon 124, 190.

Medical system 100 may include second electrode 186, second conductor 188, second balloon 190, and/or balloon body 192, and/or may define balloon recess 178, balloon recess 182, surface recess 180, surface recess 184, and/or interior volume 194, when balloon 124 and/or balloon 190 establishes any shape, and/or when fixation member 122 extends distal to distal balloon portion 164 and/or balloon 190 or distal balloon portion 164 and/or balloon 190 extends distal to fixation member 122.

As discussed, medical system 100 (e.g., medical device 108, or another external device) may include processing circuitry 125 configured to deliver therapy to and/or conduct cardiac mapping of heart 102 (FIG. 1) using conductor 148 and electrode 123. Circuitry 125 may be operably coupled to electrode 123 (e.g., via conductor 148) and second electrode 186 (e.g., via second conductor 188). Circuitry 125 may be configured to transmit therapy signals using electrode 123 and/or second electrode 186, and may be configured to receive data representative of heart 102 from electrode 123 and/or second electrode 186. In examples, circuitry 125 includes one or more processors that are configured to implement functionality and/or process instructions stored in a storage device. Processing circuitry 125 may include, for example, microprocessors, digital signal processors (DSPs), application specific integrated circuits (ASICs), field-programmable gate arrays (FPGAs), or equivalent discrete or integrated logic circuitry, or a combination of any of the foregoing devices or circuitry. Circuitry 125 may include any suitable structure, whether in hardware, software, firmware, or any combination thereof, to perform the functions ascribed herein to the processing circuitry. The functionality and/or program instructions may be embodied in software and/or firmware. The memory can include any volatile, non-volatile, magnetic, optical, or electrical media, such as a random access memory (RAM), read-only memory (ROM), non-volatile RAM (NVRAM), electrically-erasable programmable ROM (EEPROM), ferroelectric RAM (FRAM), flash memory, or any other digital media.

In examples, circuitry 125 is located within a housing of medical device 108 (FIG. 1). In other examples, circuitry 125 is located within medical device 108 and/or another device or group of devices that are not illustrated in FIG. 1. As such, techniques and capabilities attributed herein to circuitry 125 may be attributed to any combination of medical device 108 and other devices that are not illustrated in FIG. 1. Hence, medical device 108 may represent a device configured to be implanted within patient 104, extracorporeal to patient 104, any fixed or mobile computer system (e.g., a controller, a microcontroller, a personal computer, minicomputer, tablet computer, etc.), and may be generally described as including substantially all or some portion of circuitry 125.

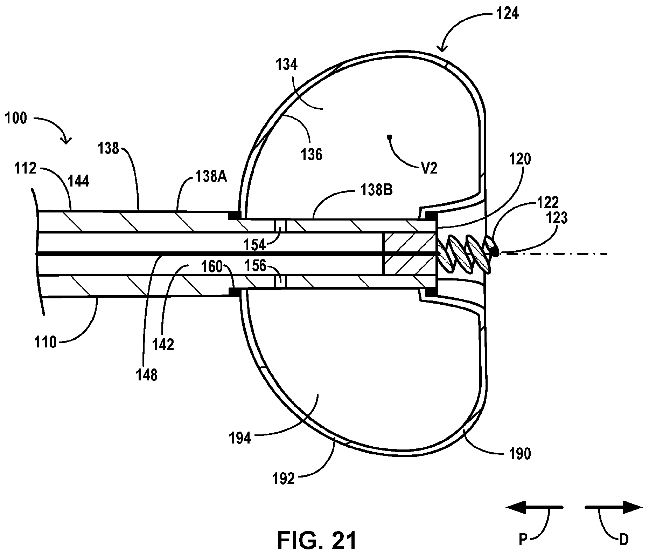

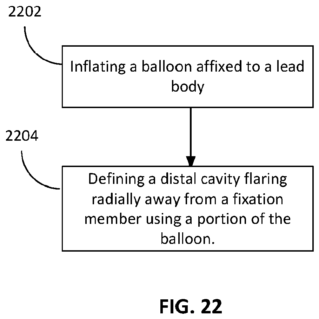

A technique for positioning an lead body 110 within a patient 104 is illustrated in FIG. 22. Although the technique is described mainly with reference to medical system 100 of FIGS. 1-21, the technique may be applied to other medical systems in other examples.

The technique includes inflating a balloon 124, 190 affixed to a lead body 110 from a deflated configuration to an inflated configuration (2202). Balloon 124, 190 may be affixed to a lead body distal portion 112 of lead body 110. The technique may include expanding balloon 124, 190 radially outward from an exterior surface 138 of lead body distal portion 112 when balloon 124, 190 inflates from the deflated configuration to the deflated configuration. In examples, the technique includes positioning lead body 110 within vasculature of patient 104 prior to inflating balloon 124, 190 from the deflated configuration to the inflated configuration.

In examples, the technique includes issuing an inflating medium into an interior volume 134 defined by balloon 124, 190 to inflate balloon 124, 190 from the deflated configuration to the inflated configuration. The technique may include issuing the inflating medium through an inflation lumen fluidly coupled to interior volume 134. Lead body 110 may define the inflation lumen. In examples, lead body 110 defines an inner lumen 142, and the technique includes issuing the inflating medium through inner lumen 142. In examples, the inflating medium issues through inner lumen 142 to interior volume 134 via side lumens 154, 156.

Balloon body 152 may extend distal to a lead distal end 120 of lead body distal portion 112 when balloon 124, 190 inflates. In examples, balloon body 152 is affixed to exterior surface 138 at a location on exterior surface 138, and a portion of balloon body displaces from a position proximal to the location to a position distal to the location when balloon 124, 190 inflates. In some examples, balloon body 152 is affixed to exterior surface 138 at a first location and affixed to exterior surface 138 at a second location distal to the first location, and a distal balloon portion 164 is distal to the first location and proximal to the second location when balloon 124, 190 is in the deflated configuration. Distal balloon portion 164 may extend distal to lead distal end 120 when balloon 124, 190 inflates.

The technique may include contacting the inflating medium and a first surface area 168 defined by a proximal balloon portion 162 and contacting the inflating medium and a second surface area 170 defined by distal balloon portion 164. First surface area 168 may define an area greater than an area defined by second surface area 170. The inflating medium may exert a greater force on first surface area 168 than on second surface area 170. The greater force on first surface area 168 may cause distal balloon portion 164 to displace distal to lead distal end 120 when balloon 124, 190 inflates to the inflated configuration. The technique may include contacting the inflating medium and a portion of balloon inner surface 136 defining a first inner dimension C1 and contacting the inflating medium and a portion of balloon inner surface 136 defining a second inner dimension C2. The technique may include contacting the inflating medium and a portion of balloon body 152 defining a first thickness T1 and contacting the inflating medium and portion of balloon body 152 defining a second thickness T2. The technique may include contacting the inflating medium and a portion of balloon body 152 including a first material having a first yield strength and contacting the inflating medium and portion of balloon body 152 including a second material having a second yield strength.

The technique may include causing balloon 124, 190 to expand from an initial dimension to an expanded dimension when balloon 124, 190 inflates. The initial dimension and the expanded dimension may be perpendicular to a longitudinal axis L defined by lead body 110. In examples, the technique includes expanding balloon 124, 190 to the expanded dimension such that a blood flow within patient 104 imparts a force on balloon 124, 190 in the direction of the blood flow, such that the expanded balloon 124, 190 acts as a sail. Balloon 124, 190 may transmit the force to lead body 110 (e.g., lead body distal portion 112) to assist in transiting lead body 110 through vasculature and/or a heart chamber of patient 104. In examples, the expanded dimension is greater than at least twenty times the initial dimension.

The technique includes defining, using a portion of an balloon outer surface 172 (e.g., cavity outer surface 181), a distal cavity 157 flaring radially away from at least some portion of fixation member 122 when balloon 124, 190 expands radially outward (2204). The portion of balloon outer surface 172 may flare radially away from fixation member 122 when the portion of outer surface 172 extends distally beyond lead distal end 120 of lead body 110. In examples, the portion of outer surface 172 increases a radial displacement from longitudinal axis L to the portion of outer surface 172 when the portion of outer surface 172 flares radially away from fixation member 122. In examples, a supporting surface 177 contacts contact outer surface 179 of balloon 124, 190 when balloon outer surface 172 (e.g., cavity outer surface 181) defines distal cavity 157.

In examples, the technique includes extending balloon 124, 190 distal to a fixation member 122 mechanically supported by lead body distal portion 112 when balloon 124, 190 is in the inflated configuration. In examples, the technique includes extending fixation member 122 distally beyond balloon 124, 190 when balloon 124, 190 is in the inflated configuration . Fixation member 122 may define a helix including a helix distal end, with the helix distal end extending distally beyond fixation member 122 when balloon 124, 190 is in the inflated configuration. Balloon 124, 190 may extend radially around a portion of fixation member 122 when balloon 124, 190 defines distal cavity 157.. In examples, balloon 124, 190 substantially surrounds a portion of fixation member 122 when balloon 124, 190 defines distal cavity 157.

Fixation member 122 may mechanically support an electrode 123 in electrical communication with processing circuitry 125 of a medical device 108. Fixation member 122 may mechanically support electrode 123 such that electrode 123 electrically communicates with tissue of patient 104 when balloon 124, 190 is in the inflated configuration. In examples, the technique includes evaluating target site 114, 116 using electrode 123 when balloon 124, 190 is in the inflated configuration. In examples, the technique includes deflating balloon 124, 190 from the inflated configuration to the deflated configuration to cause balloon 124, 190 to re-establish the initial dimension. The technique may include securing lead body 110 to tissue of patient 104 when balloon 124, 190 re-establishes the initial dimension.

In examples, the technique includes translating lead body 110 through a lumen defined by a sheath 128 when balloon 124, 190 is in the deflated configuration. Lead body distal portion 112 may be transited through sheath opening 130. Lead body distal portion 112 may transit through sheath opening 130 into the vasculature of patient 104. Lead body distal portion 112 is transited through sheath opening 130 until a marker 132 proximal to balloon 124, 190 is distal to sheath opening 130. Marker 132 may be visible on an imaging system, such as a fluoroscope, ultrasound, or other systems configured to provide images of anatomy within patient 104. In examples, balloon 124, 190 is inflated from the deflated configuration to the inflated configuration when marker 132 is distal to sheath opening 130. The invention is defined by claim 1. Preferred embodiments are defined in the dependent claims.

## Claims

1. A medical lead system (100) comprising:
an elongated lead body (110) including a distal end (120), wherein the lead body is configured to extend through vasculature of a patient;
a fixation member (122) configured to extend distal to the distal end (120); and
a balloon (124) affixed to an exterior surface (138) of the lead body (110) and defining a deflated configuration and an inflated configuration,
wherein the balloon (124) is configured to expand radially outward from the exterior surface (138) and extend distally beyond the distal end (120) of the lead body (110) when the balloon inflates from the deflated configuration to the inflated configuration,
wherein a portion of an outer surface (172) of the balloon (124) is configured to define a distal cavity (157) surrounding at least some portion of the fixation member (122) when the balloon expands radially outward, and
wherein the portion of the outer surface (172) is configured to flare radially away from the fixation member (122) as the portion of the outer surface extends distally beyond the distal end (120) of the lead body (110).

2. The medical lead system (100) of claim 1, wherein the fixation member (122) is configured to extend distal to the balloon (124) when the balloon is in the inflated configuration.

3. The medical lead system (100) of claim 1, wherein the balloon (124) is configured to extend distal to the fixation member (122) when the balloon is in the inflated configuration.

4. The medical lead system (100) of claim 1, 2 or 3,
wherein the balloon (124) includes a balloon body (152) affixed to the exterior surface (138) at a first location and affixed to the exterior surface (138) at a second location distal to the first location,
wherein the balloon includes a balloon portion (162, 163, 164) configured to be distal to the first location (160) and proximal to the second location (158) when the balloon is in the deflated configuration, and
wherein the balloon portion (162, 163, 164) is configured to extend distal to the second location (158) and beyond the distal end (120) when the balloon inflates from the deflated configuration to the inflated configuration.

5. The medical lead system (100) of claim 1, 2 or 3, wherein the balloon (124) defines an interior volume (134) configured to contain an inflating medium when the balloon inflates, and wherein the interior volume (134) is configured to define a first center of volume in the deflated configuration and a second center of volume in the inflated configuration, wherein the second center of volume is distal to the first center of volume.

6. The medical lead system (100) of claim 1, 2 or 3, wherein the balloon (124) is configured to extend radially around a portion of the fixation member (122) when the balloon is in the inflated configuration.

7. The medical lead system of claim 1, 2 or 3, wherein the balloon (124) is configured to substantially surround the portion of the fixation member (122) when the balloon is in the inflated configuration.

8. The medical system (100) of claim 1, 2 or 3, wherein the lead body defines a longitudinal axis extending through the distal end (120) and at least a portion of the fixation member (122), and wherein the portion of the outer surface (172) is configured to increase a radial displacement from the longitudinal axis to the portion of the outer surface (172) when the portion of the outer surface (172) flares radially away from the fixation member (122).

9. The medical system (100) of claim 1, 2 or 3,
wherein the lead body (110) defines a longitudinal axis (L) extending through the distal end (120),
wherein the balloon (124) includes a balloon body (152) defining a proximal balloon portion (162) and defining a distal balloon portion (164) distal to the proximal balloon portion (162),
wherein the proximal balloon portion (162) is configured to radially expand away from the longitudinal axis at a first expansion rate when an inflating medium exerts a pressure on the proximal balloon portion (162),
wherein the distal balloon portion (164) is configured to radially expand away from the longitudinal axis at a second expansion rate when the inflating medium exerts the pressure on the distal balloon portion (164), and
wherein the first expansion rate is greater than the second expansion rate.

10. The medical lead system (100) of claim 1, 2 or 3;
wherein the balloon (124) includes a balloon body (152) affixed to the exterior surface (138) at a fixation location, and
wherein balloon (124) is configured such that a portion of the balloon body (152) is proximal to the fixation location when the balloon is in the deflated configuration and the portion of the balloon body (152) is distal to the fixation location when the balloon is in the inflated configuration.

11. The medical system (100) of claim 10, wherein the fixation location is proximal to the distal end (120), and wherein the exterior surface (138) defines a supporting area (177) between the fixation location and the distal end (120), wherein the supporting area (177) is configured to contact the portion of the balloon body (152) when the balloon is in the inflated configuration.

12. The medical system (100) of claim 10, wherein the balloon body (152) is folded at the fixation location such that a first portion (173) of the balloon body (152) is positioned between a second portion (175) of the balloon body (152) and the exterior surface (138).

13. The medical lead system (100) of claim 1, 2 or 3, further comprising:
an electrode (123) supported by the fixation member (122); and
processing circuitry (125) electrically connected to the electrode (123), wherein the processing circuitry (125) is configured to deliver therapy to a tissue wall (140) using the electrode (123) when the outer surface (172) of the balloon (124) defines the distal cavity (157).

14. The medical lead system (100) of claim 13, wherein the processing circuitry (125) is configured to deliver therapy to the tissue wall (140) when the balloon (124) extends distal to the fixation element (122), a fluid substantially fills the distal cavity (157) and fluidically couples the electrode (123) and the tissue wall (140), and the electrode (123) is displaced from the tissue wall (140).

15. The medical lead system (100) of claim 1, 2 or 3, wherein the balloon (124) is a first balloon and the medical lead system further comprises a second balloon (190),
wherein the second balloon (190) is affixed to the exterior surface (138) of the lead body (110) and defines a second deflated figuration and a second inflated configuration,
wherein the second balloon (190) is configured to expand radially outward from the exterior surface (138) and extend distally beyond the distal end (120) of the lead body (110) when the second balloon (190) inflates from the second deflated configuration to the second inflated configuration, and
wherein the fixation member (122) is configured to extend distal to the second balloon (190) when the second balloon (190) is in the inflated configuration.

## Patentansprüche

1. Medizinisches Leitungssystem (100), umfassend:
einen länglichen Leitungskörper (110), der ein distales Ende (120) einschließt, wobei der Leitungskörper konfiguriert ist, um sich durch ein Gefäßsystem eines Patienten zu erstrecken;
ein Fixierungselement (122), das konfiguriert ist, um sich distal zu dem distalen Ende (120) zu erstrecken; und
einen Ballon (124), der an einer Außenoberfläche (138) des Leitungskörpers (110) befestigt ist und eine entleerte Konfiguration und eine aufgeblasene Konfiguration definiert,
wobei der Ballon (124) konfiguriert ist, um sich von der Außenoberfläche (138) radial nach außen zu expandieren und sich distal über das distale Ende (120) des Leitungskörpers (110) hinaus zu erstrecken, wenn sich der Ballon von der entleerten Konfiguration in die aufgeblasene Konfiguration aufbläst,
wobei ein Abschnitt einer Außenoberfläche (172) des Ballons (124) konfiguriert ist, um einen distalen Hohlraum (157) zu definieren, der mindestens einen Abschnitt des Fixierungselements (122) umgibt, wenn der Ballon radial nach außen expandiert, und
wobei der Abschnitt der Außenoberfläche (172) konfiguriert ist, um sich radial von dem Fixierungselement (122) weg aufzuweiten, wenn sich der Abschnitt der Außenoberfläche distal über das distale Ende (120) des Leitungskörpers (110) hinaus erstreckt.

2. Medizinisches Leitungssystem (100) nach Anspruch 1, wobei das Fixierungselement (122) konfiguriert ist, um sich distal zu dem Ballon (124) zu erstrecken, wenn der Ballon in der aufgeblasenen Konfiguration ist.

3. Medizinisches Leitungssystem (100) nach Anspruch 1, wobei der Ballon (124) konfiguriert ist, um sich distal zu dem Fixierungselement (122) zu erstrecken, wenn der Ballon in der aufgeblasenen Konfiguration ist.

4. Medizinisches Leitungssystem (100) nach einem der Ansprüche 1, 2 oder 3,
wobei der Ballon (124) einen Ballonkörper (152) einschließt, der an der Außenoberfläche (138) an einer ersten Stelle befestigt ist und an der Außenoberfläche (138) an einer zweiten Stelle distal zu der ersten Stelle befestigt ist,
wobei der Ballon einen Ballonabschnitt (162, 163, 164) einschließt, der konfiguriert ist, um distal zu der ersten Stelle (160) und proximal zu der zweiten Stelle (158) zu liegen, wenn der Ballon in der entleerten Konfiguration ist, und
wobei der Ballonabschnitt (162, 163, 164) konfiguriert ist, um sich distal zu der zweiten Stelle (158) und über das distale Ende (120) hinaus zu erstrecken, wenn der Ballon von der entleerten Konfiguration in die aufgeblasene Konfiguration aufgeblasen wird.

5. Medizinisches Leitungssystem (100) nach Anspruch 1, 2 oder 3, wobei der Ballon (124) ein Innenvolumen (134) definiert, das konfiguriert ist, um ein Aufblasmedium zu enthalten, wenn der Ballon aufgeblasen wird, und wobei das Innenvolumen (134) konfiguriert ist, um einen ersten Volumenmittelpunkt in der entleerten Konfiguration und einen zweiten Volumenmittelpunkt in der aufgeblasenen Konfiguration zu definieren, wobei der zweite Volumenmittelpunkt distal zu dem ersten Volumenmittelpunkt liegt.

6. Medizinisches Leitungssystem (100) nach Anspruch 1, 2 oder 3, wobei der Ballon (124) konfiguriert ist, um sich radial um einen Abschnitt des Fixierungselements (122) zu erstrecken, wenn der Ballon in der aufgeblasenen Konfiguration ist.

7. Medizinisches Leitungssystem nach Anspruch 1, 2 oder 3, wobei der Ballon (124) konfiguriert ist, um den Abschnitt des Fixierungselements (122) im Wesentlichen zu umgeben, wenn der Ballon in der aufgeblasenen Konfiguration ist.

8. Medizinisches System (100) nach Anspruch 1, 2 oder 3, wobei der Leitungskörper eine Längsachse definiert, die sich durch das distale Ende (120) und mindestens einen Abschnitt des Fixierungselements (122) erstreckt, und wobei der Abschnitt der Außenoberfläche (172) konfiguriert ist, um eine radiale Verschiebung von der Längsachse zu dem Abschnitt der Außenoberfläche (172) zu vergrößern, wenn sich der Abschnitt der Außenoberfläche (172) radial von dem Fixierungselement (122) weg aufweitet.

9. Medizinisches System (100) nach einem der Ansprüche 1, 2 oder 3,
wobei der Leitungskörper (110) eine Längsachse (L) definiert, die sich durch das distale Ende (120) erstreckt,
wobei der Ballon (124) einen Ballonkörper (152) einschließt, der einen proximalen Ballonabschnitt (162) definiert und einen distalen Ballonabschnitt (164) distal zu dem proximalen Ballonabschnitt (162) definiert,
wobei der proximale Ballonabschnitt (162) konfiguriert ist, um sich mit einer ersten Expansionsrate radial von der Längsachse weg zu expandieren, wenn ein Aufblasmedium einen Druck auf den proximalen Ballonabschnitt (162) ausübt,
wobei der distale Ballonabschnitt (164) konfiguriert ist, um sich mit einer zweiten Expansionsrate radial von der Längsachse weg zu expandieren, wenn das Aufblasmedium den Druck auf den distalen Ballonabschnitt (164) ausübt, und
wobei die erste Expansionsrate größer als die zweite Expansionsrate ist.

10. Medizinisches Leitungssystem (100) nach einem der Ansprüche 1, 2 oder 3;
wobei der Ballon (124) einen Ballonkörper (152) einschließt, der an der Außenoberfläche (138) an einer Fixierungsstelle befestigt ist, und
wobei der Ballon (124) derart konfiguriert ist, dass ein Abschnitt des Ballonkörpers (152) proximal zu der Fixierungsstelle ist, wenn der Ballon in der entleerten Konfiguration ist, und der Abschnitt des Ballonkörpers (152) distal zu der Fixierungsstelle ist, wenn der Ballon in der inflatierten Konfiguration ist.

11. Medizinisches System (100) nach Anspruch 10, wobei die Fixierungsstelle proximal zu dem distalen Ende (120) liegt, und wobei die Außenoberfläche (138) einen Tragbereich (177) zwischen der Fixierungsstelle und dem distalen Ende (120) definiert, wobei der Tragbereich (177) konfiguriert ist, um den Abschnitt des Ballonkörpers (152) zu kontaktieren, wenn der Ballon in der aufgeblasenen Konfiguration ist.

12. Medizinisches System (100) nach Anspruch 10, wobei der Ballonkörper (152) an der Fixierungsstelle derart gefaltet ist, dass ein erster Abschnitt (173) des Ballonkörpers (152) zwischen einem zweiten Abschnitt (175) des Ballonkörpers (152) und der Außenoberfläche (138) positioniert ist.

13. Medizinisches Leitungssystem (100) nach Anspruch 1, 2 oder 3, ferner umfassend:
eine Elektrode (123), die durch das Fixierungselement (122) getragen wird; und
einen Verarbeitungsschaltkreis (125), der mit der Elektrode (123) elektrisch verbunden ist, wobei der Verarbeitungsschaltkreis (125) konfiguriert ist, um unter Verwendung der Elektrode (123) eine Therapie an eine Gewebewand (140) abzugeben, wenn die Außenoberfläche (172) des Ballons (124) den distalen Hohlraum (157) definiert.

14. Medizinisches Leitungssystem (100) nach Anspruch 13, wobei der Verarbeitungsschaltkreis (125) konfiguriert ist, um die Therapie an die Gewebewand (140) abzugeben, wenn sich der Ballon (124) distal zu dem Fixierungselement (122) erstreckt, ein Fluid den distalen Hohlraum (157) im Wesentlichen füllt und die Elektrode (123) und die Gewebewand (140) fluidisch koppelt, und die Elektrode (123) von der Gewebewand (140) versetzt ist.

15. Medizinisches Leitungssystem (100) nach Anspruch 1, 2 oder 3, wobei der Ballon (124) ein erster Ballon ist und das medizinische Leitungssystem ferner einen zweiten Ballon (190) umfasst,
wobei der zweite Ballon (190) an der Außenoberfläche (138) des Leitungskörpers (110) befestigt ist und eine zweite entleerte Konfiguration und eine zweite aufgeblasene Konfiguration definiert,
wobei der zweite Ballon (190) konfiguriert ist, um sich von der Außenoberfläche (138) radial nach außen zu expandieren und sich distal über das distale Ende (120) des Leitungskörpers (110) hinaus zu erstrecken, wenn sich der zweite Ballon (190) von der zweiten entleerten Konfiguration in die zweite inflatierte Konfiguration aufbläst, und
wobei das Fixierungselement (122) konfiguriert ist, um sich distal zu dem zweiten Ballon (190) zu erstrecken, wenn der zweite Ballon (190) in der aufgeblasenen Konfiguration ist.

## Revendications

1. Système de dérivation médicale (100) comprenant :
un corps de dérivation (110) allongé comportant une extrémité distale (120), dans lequel le corps de dérivation est conçu pour s'étendre à travers le système vasculaire d'un patient ;
un élément de fixation (122) conçu pour s'étendre distalement par rapport à l'extrémité distale (120) ; et
un ballonnet (124) fixé à une surface extérieure (138) du corps de dérivation (110) et définissant une configuration dégonflée et une configuration gonflée,
dans lequel le ballonnet (124) est conçu pour s'expanser radialement vers l'extérieur à partir de la surface extérieure (138) et s'étendre distalement au-delà de l'extrémité distale (120) du corps de dérivation (110) lorsque le ballonnet se gonfle de la configuration dégonflée à la configuration gonflée,
dans lequel une partie d'une surface externe (172) du ballonnet (124) est conçue pour définir une cavité distale (157) entourant au moins une certaine partie de l'élément de fixation (122) lorsque le ballonnet s'expanse radialement vers l'extérieur, et
dans lequel la partie de la surface externe (172) est conçue pour s'évaser radialement à l'écart de l'élément de fixation (122) à mesure que la partie de la surface externe s'étend distalement au-delà de l'extrémité distale (120) du corps de dérivation (110).

2. Système de dérivation médicale (100) selon la revendication 1, dans lequel l'élément de fixation (122) est conçu pour s'étendre distalement par rapport au ballonnet (124) lorsque le ballonnet est dans la configuration gonflée.

3. Système de dérivation médicale (100) selon la revendication 1, dans lequel le ballonnet (124) est conçu pour s'étendre distalement par rapport à l'élément de fixation (122) lorsque le ballonnet est dans la configuration gonflée.

4. Système de dérivation médicale (100) selon la revendication 1, 2 ou 3,
dans lequel le ballonnet (124) comporte un corps de ballonnet (152) fixé à la surface extérieure (138) au niveau d'une première localisation et fixé à la surface extérieure (138) au niveau d'une seconde localisation distale par rapport à la première localisation,
dans lequel le ballonnet comporte une partie de ballonnet (162, 163, 164) conçue pour être distale par rapport à la première localisation (160) et proximale par rapport à la seconde localisation (158) lorsque le ballonnet est dans la configuration dégonflée, et
dans lequel la partie de ballonnet (162, 163, 164) est conçue pour s'étendre distalement par rapport à la seconde localisation (158) et au-delà de l'extrémité distale (120) lorsque le ballonnet se gonfle de la configuration dégonflée à la configuration gonflée.

5. Système de dérivation médicale (100) selon la revendication 1, 2 ou 3, dans lequel le ballonnet (124) définit un volume intérieur (134) conçu pour contenir un milieu de gonflage lorsque le ballonnet se gonfle, et dans lequel le volume intérieur (134) est conçu pour définir un premier centre de volume dans la configuration dégonflée et un second centre de volume dans la configuration gonflée, dans lequel le second centre de volume est distal par rapport au premier centre de volume.

6. Système de dérivation médicale (100) selon la revendication 1, 2 ou 3, dans lequel le ballonnet (124) est conçu pour s'étendre radialement autour d'une partie de l'élément de fixation (122) lorsque le ballonnet est dans la configuration gonflée.

7. Système de dérivation médicale selon la revendication 1, 2 ou 3, dans lequel le ballonnet (124) est conçu pour entourer sensiblement la partie de l'élément de fixation (122) lorsque le ballonnet est dans la configuration gonflée.

8. Système médical (100) selon la revendication 1, 2 ou 3, dans lequel le corps de dérivation définit un axe longitudinal s'étendant à travers l'extrémité distale (120) et au moins une partie de l'élément de fixation (122), et dans lequel la partie de la surface externe (172) est conçue pour augmenter un déplacement radial allant de l'axe longitudinal à la partie de la surface externe (172) lorsque la partie de la surface externe (172) s'évase radialement à l'écart de l'élément de fixation (122).

9. Système médical (100) selon la revendication 1, 2 ou 3,
dans lequel le corps de dérivation (110) définit un axe longitudinal (L) s'étendant à travers l'extrémité distale (120),
dans lequel le ballonnet (124) comporte un corps de ballonnet (152) définissant une partie proximale de ballonnet (162) et définissant une partie distale de ballonnet (164) distale par rapport à la partie proximale de ballonnet (162),
dans lequel la partie proximale de ballonnet (162) est conçue pour s'expanser radialement à l'écart de l'axe longitudinal à un premier taux d'expansion lorsqu'un milieu de gonflage exerce une pression sur la partie proximale de ballonnet (162),
dans lequel la partie distale de ballonnet (164) est conçue pour s'expanser radialement à l'écart de l'axe longitudinal à un second taux d'expansion lorsque le milieu de gonflage exerce la pression sur la partie distale de ballonnet (164), et
dans lequel le premier taux d'expansion est supérieur au second taux d'expansion.

10. Système de dérivation médicale (100) selon la revendication 1, 2 ou 3 ;
dans lequel le ballonnet (124) comporte un corps de ballonnet (152) fixé à la surface extérieure (138) au niveau d'une localisation de fixation, et
dans lequel le ballonnet (124) est conçu de telle sorte qu'une partie du corps de ballonnet (152) est proximale par rapport à la localisation de fixation lorsque le ballonnet est dans la configuration dégonflée et la partie du corps de ballonnet (152) est distale par rapport à la localisation de fixation lorsque le ballonnet est dans la configuration gonflée.

11. Système médical (100) selon la revendication 10, dans lequel la localisation de fixation est proximale par rapport à l'extrémité distale (120), et dans lequel la surface extérieure (138) définit une zone de support (177) entre la localisation de fixation et l'extrémité distale (120), dans lequel la zone de support (177) est conçue pour venir en contact avec la partie du corps de ballonnet (152) lorsque le ballonnet est dans la configuration gonflée.

12. Système médical (100) selon la revendication 10, dans lequel le corps de ballonnet (152) est plié au niveau de la localisation de fixation de telle sorte qu'une première partie (173) du corps de ballonnet (152) est positionnée entre une seconde partie (175) du corps de ballonnet (152) et la surface extérieure (138).

13. Système de dérivation médicale (100) selon la revendication 1, 2 ou 3, comprenant en outre :
une électrode (123) supportée par l'élément de fixation (122) ; et
un système de circuits de traitement (125) connecté électriquement à l'électrode (123), dans lequel le système de circuits de traitement (125) est configuré pour délivrer une thérapie à une paroi tissulaire (140) à l'aide de l'électrode (123) lorsque la surface externe (172) du ballonnet (124) définit la cavité distale (157).

14. Système de dérivation médicale (100) selon la revendication 13, dans lequel le système de circuits de traitement (125) est configuré pour délivrer une thérapie à la paroi tissulaire (140) lorsque le ballonnet (124) s'étend distalement par rapport à l'élément de fixation (122), un fluide remplit sensiblement la cavité distale (157) et couple fluidiquement l'électrode (123) et la paroi tissulaire (140), et l'électrode (123) est déplacée par rapport à la paroi tissulaire (140).

15. Système de dérivation médicale (100) selon la revendication 1, 2 ou 3, dans lequel le ballonnet (124) est un premier ballonnet et le système de dérivation médicale comprend en outre un second ballonnet (190),
dans lequel le second ballonnet (190) est fixé à la surface extérieure (138) du corps de dérivation (110) et définit une seconde configuration dégonflée et une seconde configuration gonflée,
dans lequel le second ballonnet (190) est conçu pour s'expanser radialement vers l'extérieur à partir de la surface extérieure (138) et s'étendre distalement au-delà de l'extrémité distale (120) du corps de dérivation (110) lorsque le second ballonnet (190) se gonfle de la seconde configuration dégonflée à la seconde configuration gonflée, et
dans lequel l'élément de fixation (122) est conçu pour s'étendre distalement par rapport au second ballonnet (190) lorsque le second ballonnet (190) est dans la configuration gonflée.
